# EUROPÄISCHE PATENTSCHRIFT

(11) **EP 3 851 081 B1**
(45) Veröffentlichungstag und Bekanntmachung des Hinweises auf die Patenterteilung: **13.04.2022**
(21) Anmeldenummer: 21151555.6
(22) Anmeldetag: 14.01.2021
(51) Int. Cl.: A61F 5/01

(54) **GELENKANORDNUNG FÜR EINE ORTHESE EINES EXTREMITÄTENGELENKS UND ORTHESE MIT EINER DERARTIGEN GELENKANORDNUNG**
ARTICULATED ARRANGEMENT FOR AN ORTHESIS OF AN EXTREMITY JOINT AND ORTHOSIS WITH SUCH AN ARTICULATED ARRANGEMENT
AGENCEMENT ARTICULAIRE POUR UNE ORTHÈSE D'UNE ARTICULATION D'EXTRÉMITÉ ET ORTHÈSE DOTÉE D'UN TEL AGENCEMENT ARTICULAIRE

(30) Priorität: 16.01.2020 DE 102020000254
(43) Veröffentlichungstag der Anmeldung: 21.07.2021
(73) Patentinhaber: Bort GmbH, 71384 Weinstadt-Benzach (DE)
(72) Erfinder: Scheuermann, Rainer, 24223 Raisdorf (DE)
(74) Vertreter: Thum, Bernhard

(56) Entgegenhaltungen:
- EP-A2- 1 642 554
- EP-B1- 2 524 672
- US-A1- 2005 148 916

## Beschreibung

Die vorliegende Erfindung betrifft eine Gelenkanordnung für eine Orthese eines Extremitätengelenks und eine Orthese umfassend eine derartige Gelenkanordnung.

Orthesen werden vordergründig dazu eingesetzt, Regionen eines Körpers eines Patienten zu stabilisieren, zu entlasten, zu führen oder zu korrigieren. Dies kann therapeutisch im Rahmen einer Behandlung einer diagnostizierten Erkrankung des Patienten oder auch zur prophylaktischen Unterstützung des Patienten erfolgen. Im Falle einer Orthese, die am Körper eines Patienten einer Bewegung des Körpers ausgesetzt ist, wie beispielsweise einer Orthese für ein Extremitätengelenk, ist es vorteilhaft, wenn die eingesetzte Orthese einem natürlichen physiologischen Bewegungsablauf des Körpers bzw. des Extremitätengelenks folgt. Allerdings können Extremitätengelenke komplexe natürliche Bewegungsabläufe umfassen. Derart komplexe Bewegungsabläufe liegen insbesondere dann vor, wenn der natürliche Bewegungsablauf des Extremitätengelenks keine feste Drehachse aufweist, um die sich das Extremitätengelenk dreht. Entsprechend schwierig ist es, einen solchen komplexen Bewegungsablauf mit der Orthese nachzubilden.

Orthesen an einem Extremitätengelenk umfassen Befestigungsmittel, mit denen die Orthese an dem Extremitätengelenk bzw. das Extremitätengelenk überbrückend an dem Patienten befestigt werden kann. Bildet die Orthese des Extremitätengelenks allerdings nicht den natürlichen Bewegungsablauf nach, was in der Regel bei genannten komplexen Bewegungsabläufen der Fall ist, so führt dies zu Nachteilen für den Patienten. Die Befestigungsmittel der Orthese können sich relativ zu dem Patienten und insbesondere relativ zur Haut des Patienten bewegen, was zu unangenehmen Hautreizungen des Patienten führen kann. Ferner kann die Orthese selbst eine unerwünschte Migration am Körper des Patienten vollziehen. Insgesamt kann sich somit für einen Patienten ein unangenehmer Tragekomfort einstellen.

Auch kann eine Orthese, die nicht den natürlichen Bewegungsablauf des Extremitätengelenks nachbildet, dem Extremitätengelenk einen Bewegungsablauf induzieren, der nicht natürlich ist. Dies kann zu einer Beeinträchtigung bis hin zu einer verstärkten Abnutzung des Extremitätengelenks oder auch zu einem unangenehmen Tragekomfort führen. Ferner kann dies weitere Krankheitsbilder begünstigen.

Auch kann es im Rahmen von therapeutischen oder prophylaktischen Ansätzen gerade erforderlich sein, dass der Bewegungsablauf der Orthese der natürlichen Gelenkbewegung des Extremitätengelenks folgt.

Ein komplexer Bewegungsablauf liegt beispielsweise im menschlichen Kniegelenk vor, denn es vollzieht zumindest phasenweise eine kombinierte Roll- und Gleitbewegung. Die kombinierte Roll- und Gleitbewegung betrifft im technischen Sinne Drehbewegungen eines Körpers. Eine Drehbewegung ist jedoch als Drehung eines Körpers um eine feststehende Drehachse spezifiziert. Eine Rollbewegung ist hingegen eine Drehung um eine sich bewegende Drehachse. So ist eine Rollbewegung vergleichbar mit einem Rad, das auf einer Ebene abrollt. Das Zentrum des Rades bewegt sich in der Richtung der Rollbewegung. Eine Gleitbewegung ist vergleichbar mit einem Rad, das auf der Ebene abrutscht. Das Zentrum des Rades verändert sich gegenüber der Ebene nicht. Im Fall der kombinierten Roll- und Gleitbewegung findet demnach eine Bewegung statt, bei der das Rad zwar auf der Ebene abrollt, sich jedoch die Drehachse um einen kleineren oder höheren Betrag in Richtung der Bewegung bewegt, als dies durch die Rollbewegung induziert wäre.

Im Stand der Technik sind Versuche bekannt, komplexe Bewegungsabläufe von Extremitätengelenken besser nachzubilden. So ist beispielsweise aus dem Dokument EP 2 524 672 B1 eine Gelenkanordnung für eine Knieorthese bekannt. Die Gelenkanordnung umfasst einen Führungskörper, der mit einem ersten Gelenkschenkel verbunden ist, und eine Ausnehmung, die mit einem zweiten Gelenkschenkel verbunden ist. Der Führungskörper ist beispielsweise in Form eines Dreiecks ausgebildet und ist derart in der zugehörigen Ausnehmung geführt, die drei Führungskurven aufweist, dass er in der Ausnehmung und entlang der Führungskurven gleitet.

Die aus dem Stand der Technik bekannten Orthesen mit einer Gelenkanordnung, die einen Bewegungsablauf mit einer sich ändernden Drehachse nachbilden sollen, sind allerdings mit Nachteilen verbunden. Trotz vieler Bemühungen wird es nicht bzw. allenfalls ansatzweise erreicht, den natürlichen physiologischen Bewegungsablauf des Extremitätengelenks tatsächlich nachzubilden. Häufig müssen teils wesentliche Vereinfachungen des natürlichen Bewegungsablaufs in Kauf genommen werden. Damit sind zumindest Phasen des Bewegungsablaufs unzureichend durch die Gelenkanordnung nachgebildet. Auch besteht das Problem, dass bekannte Gelenkanordnungen häufig mechanisch nicht ausreichend stabil ausgebildet sind. Dies führt zu einer nur ungenauen Unterstützung des Extremitätengelenks und zu einem unsicheren Tragegefühl. Ferner weisen bekannte Gelenkanordnungen eine Vielzahl an Komponenten auf, was zu einer aufwändigen Fertigung führt.

Zum weiteren Stand der Technik wird auf die Dokumente AT 510 403 A4, US 3 817 244 A und US 5 244 455 A verwiesen. In all diesen Dokumenten wird das Bewegungsprofil der "Rastpolkurve" beschrieben, bzw. konstruktiv nachempfunden. Damit können diese Orthesen-Gelenke lediglich die Längendifferenz und den Parallelverschub von Orthesen-Gelenk-Schienen einigermaßen ausgleichen, was allerdings auch von der jeweiligen Größe und Ausprägung des Gelenkmimik und der Gestaltung der Orthese selbst abhängig ist. Die vorgestellten Gelenkkonstruktionen können allerdings nicht einem sog. "Schubladen-Effekt" im Kniegelenk entgegenwirken, wie das bei dem Polkurvengelenk der Fall ist, weil hier die spezielle Ausprägung und ein permanent geführter Kontakt, an mindestens drei Stellen, fehlt und dadurch sogar in der Beugung die Gelenke sperren können. Dabei kommt es insbesondere häufig während der Beugebewegung zu einer Translationsverschiebung, die sich von dem üblichen physiologischen Bewegungsablauf eines solchen Extremitätengelenks, beispielsweise eines Kniegelenks, unterscheidet.

Eine Aufgabe der vorliegenden Erfindung ist es daher, eine verbesserte Gelenkanordnung für eine Orthese eines Extremitätengelenks bereitzustellen. Insbesondere ist es eine Aufgabe der vorliegenden Erfindung, eine gegenüber dem Stand der Technik verbesserte Gelenkanordnung für eine Orthese eines Extremitätengelenks bereitzustellen, die einen Bewegungsablauf mit sich veränderndem Drehzentrum realisiert, sodass der physiologische Bewegungsablauf des Extremitätengelenks möglichst exakt nachgebildet werden kann. Ferner ist es Aufgabe der Erfindung, eine verbesserte Orthese mit einer Gelenkanordnung bereitzustellen.

Die erfindungsgemäße Aufgabe wird durch eine Gelenkanordnung für eine Orthese eines Extremitätengelenks mit den Merkmalen des Anspruchs 1 gelöst. Ferner wird die erfindungsgemäße Aufgabe durch eine Orthese mit den Merkmalen des Anspruchs 15 gelöst. Vorteilhafte Weiterbildungen der Erfindung sind in den Unteransprüchen beschrieben.

Die oben genannte Aufgabe wird gemäß Anspruch 1 durch eine Gelenkanordnung für eine Orthese eines Extremitätengelenks gelöst. Die Gelenkanordnung umfasst einen ersten Gelenkschenkel mit einem länglichen ersten Schenkelabschnitt und einem ersten Kopfabschnitt und einen zweiten Gelenkschenkel mit einem länglichen zweiten Schenkelabschnitt und einem zweiten Kopfabschnitt. Der erste und zweite Gelenkschenkel im Bereich des ersten bzw. zweiten Kopfabschnitts überlappenden sich zumindest teilweise flächig. Der erste und zweite Gelenkschenkel sind ferner derart gelenkig miteinander verbunden, dass die Gelenkschenkel in einer Schwenkebene zueinander verschwenkbar sind. In dem ersten Kopfabschnitt ist eine Ausnehmung und an dem zweiten Kopfabschnitt ein Führungskörper vorgesehen. Der Führungskörper ist in der Ausnehmung angeordnet. Ferner ist der Führungskörper relativ zu der Ausnehmung bewegbar angeordnet. Die Ausnehmung weist eine Innenkontur und der Führungskörper eine Außenkontur auf, die derart ausgebildet sind, dass während einem Verschwenken der Gelenkschenkel zueinander die Ausnehmung relativ zu dem Führungskörper eine Rotations- und Translationsbewegung ausführt, bei der sie um den Führungskörper zwangsgeführt ist und zumindest phasenweise ein vorgegebener Abschnitt der Innenkontur auf einem vorgegebenen Abschnitt der Außenkontur abrollt.

Durch die erfindungsgemäße Gelenkanordnung kann erreicht werden, dass ein vorgegebener Bewegungsablauf erzeugt wird. Der Bewegungsablauf ist dabei einfach vorzugeben, da der vorgegebene Abschnitt der Innenkontur auf dem vorgegebenen Abschnitt der Außenkontur abrollt. So kann ein natürlicher Bewegungsablauf des Extremitätengelenks einfach nachgebildet werden.

Die Zwangsführung ermöglicht es, dass die relative Bewegung zwischen Führungskörper und Ausnehmung eindeutig definiert ist. Dabei wird ein Spiel zwischen Führungskörper und Ausnehmung verhindert. Weitere Komponenten sind für eine eindeutig definierte relative Bewegung nicht notwendig. Damit kann eine genaue Bewegung der Gelenkanordnung erreicht werden.

Ferner wird durch die erfindungsgemäße Gelenkanordnung ein einfacher Aufbau erreicht, der den natürlichen Bewegungsablauf des Extremitätengelenks nachbildet, ohne dass in den Bewegungsablauf eine Vielzahl an Komponenten involviert ist.

Vorteilhafterweise kann ferner auftretende Reibung zwischen Führungskörper und Ausnehmung reduziert werden. Damit kann eine gesteigerte Lebensdauer der erfindungsgemäßen Gelenkanordnung erreicht werden.

Ferner kann erfindungsgemäß vorgesehen sein, dass der Führungskörper tropfenförmig ausgebildet ist. Somit ist die Außenkontur des Führungskörpers vergleichsweise groß ausgebildet, was eine flächige Einleitung von Kräften in den Führungskörper begünstigt. Ferner kann verbessert eine sich während der Bewegung verändernde Drehachse berücksichtigt werden.

Gemäß einer Weiterbildung der vorliegenden Erfindung kann vorgesehen sein, dass der tropfenförmige Führungskörper einen verdickten Kopfbereich und einen sich verjüngenden Fortsatz umfasst. Der verdickte Kopfbereich kann in vorteilhafter Weise große Kräfte in der Gelenkanordnung aufnehmen bzw. einleiten. Ferner verbessert der verdickte Kopfbereich die Führung des Führungskörpers relativ zu der Ausnehmung. Der sich verjüngende Fortsatz begünstigt die exakte Führung des Führungskörpers relativ zu der Ausnehmung. Ferner begünstigt der Fortsatz das Abrollen der Innenkontur auf der Außenkontur.

Gemäß einem Aspekt der Erfindung kann der Führungskörper bogenförmig und/oder harmonisch gekrümmt ausgebildet sein. Damit kann in vorteilhafter Weise das Abrollen begünstigt werden. Außerdem kann ein Führungskörper erreicht werden, der einen natürlichen Bewegungsablauf begünstigt. Ferner kann eine bogenförmige bzw. gekrümmte Form des Führungskörpers dessen Stabilität steigern.

Gemäß einer Weiterbildung der Erfindung kann vorgesehen sein, dass eine Innenseite des Führungskörpers im Wesentlichen den vorgegebenen Abschnitt der Außenkontur umfasst. Damit kann erreicht werden, dass die Innenseite des Führungskörpers relativ zu der Ausnehmung abrollt. Auftretende Reibung an der Innenseite des Führungskörpers kann dadurch reduziert werden. Außerdem kann durch eine gezielte Ausgestaltung und/oder Anpassung der Innenseite des Führungskörpers das Abrollen eingestellt bzw. angepasst werden. Ferner kann der natürliche Bewegungsablauf des Extremitätengelenks verbessert nachgebildet werden.

Eine bevorzugte Ausführungsform der Erfindung sieht vor, dass die Ausnehmung herzförmig ausgebildet ist. Somit ist die Innenkontur der Ausnehmung vergleichsweise groß ausgebildet, was eine flächige Einleitung von Kräften in die Ausnehmung begünstigt. Ferner kann besser eine sich während der Bewegung verändernde Drehachse berücksichtigt werden. Außerdem kann durch die herzförmige Ausnehmung erreich werden, dass unterschiedliche Phasen des natürlichen Bewegungsablaufs verbessert abgebildet werden.

Gemäß einer Weiterbildung kann vorgesehen sein, dass die herzförmige Ausnehmung eine erste, insbesondere distale, und eine zweite, bevorzugt proximale, Kammer umfasst, wobei ein Vorsprung zwischen den Kammern ausgebildet ist. So können unterschiedliche Phasen des natürlichen Bewegungsablaufs verbessert abgebildet werden. Insgesamt kann der natürliche Bewegungsablauf verbessert abgebildet werden.

Ferner kann erfindungsgemäß vorgesehen sein, dass der Vorsprung im Wesentlichen den vorgegebenen Abschnitt der Innenkontur umfasst. Damit kann erreicht werden, dass der Vorsprung relativ zu dem Führungskörper abrollt. Auftretende Reibung an der Innenseite der Ausnehmung kann dadurch reduziert werden. Außerdem kann durch eine gezielte Ausgestaltung und/oder Anpassung der Außenseite der Ausnehmung das Abrollen eingestellt bzw. angepasst werden. Ferner kann der natürliche Bewegungsablauf des Extremitätengelenks verbessert nachgebildet werden.

Insgesamt kann durch den tropfenförmigen Führungskörper und die herzförmige Ausnehmung erreicht werden, dass die Außenkontur des Führungskörpers und die Innenkontur der Ausnehmung aufeinander abgestimmt sind. Ferner ist der Führungskörper relativ zu der Ausnehmung zwangsgeführt.

Gemäß einer bevorzugten Ausführungsform kann vorgesehen sein, dass in einer vollständig gestreckten Stellung der Gelenkschenkel zueinander ein Anfangspunkt des vorgegebenen Abschnitts der Außenkontur einen Anfangspunkt des vorgegebenen Abschnitts der Innenkontur berührt. Vorteilhafterweise kann damit ein fester Anfangspunkt der relativen Bewegung der Ausnehmung gegenüber dem Führungskörper definiert werden. Ferner kann ein Abbilden des natürlichen Bewegungsablaufs verbessert werden.

Gemäß einer Weiterbildung der vorliegenden Erfindung kann vorgesehen sein, dass in der vollständig gestreckten Stellung der Gelenkschenkel zueinander ein Teil der Außenkontur flächig einen Teil der Innenkontur kontaktiert. Der Teil der Außenkontur kann dabei eine äußere Seite des tropfenförmigen Führungskörpers, insbesondere die Außenseite des Führungskörpers, sein. Der Teil der Innenkontur kann ferner eine äußere Seite der ersten Kammer der Ausnehmung sein. Das flächige Kontaktieren begünstigt eine Kraftübertragung zwischen Außenkontur und Innenkontur, bei der übertragene Kräfte auf eine größere Fläche verteilt werden können. So können hohe Flächenerpressungen vermieden werden. Im Hinblick auf ein gestrecktes Extremitätengelenk bedeutet dies, dass Kräfte in der gestreckten Stellung des Extremitätengelenks flächig übertragen werden können. Ferner kann eine exakt definierte Stellung der Gelenkanordnung in der vollständig gestreckten Stellung erreicht werden.

Gemäß einem weiteren optionalen Aspekt der vorliegenden Erfindung kann vorgesehen sein, dass der Führungskörper in der vollständig gestreckten Stellung der Gelenkschenkel zueinander im Wesentlichen in der ersten Kammer, insbesondere in einem unteren, bevorzugt distalen, Bereich der ersten Kammer, angeordnet ist.

Gemäß einer weiteren optionalen erfindungsgemäßen Ausgestaltung kann vorgesehen sein, dass der Führungskörper in der vollständig gestreckten Stellung derart in der ersten Kammer angeordnet ist, dass er lediglich eine Bewegung in einer einzigen Richtung relativ zu der Ausnehmung vollziehen kann. In anderen Worten bedeutet dies, dass ein weiteres Strecken bzw. ein Überstrecken der Gelenkanordnung nicht möglich ist. Die Gelenkanordnung kann ausgehend von der vollständig gestreckten Stellung lediglich ein Verschwenken in Richtung einer gebeugten Stellung vollziehen. So kann in vorteilhafter Weise erreicht werden, dass die Bewegung entsprechend einer natürlichen maximalen Streckung des Extremitätengelenks oder einer gewünschten maximalen Streckung des Extremitätengelenks begrenzt bzw. abgrenzbar ist. Eine Schädigung des Extremitätengelenks durch Überstreckung kann vermieden werden.

Gemäß einer bevorzugten Ausführungsform kann vorgesehen sein, dass in einer vollständig gebeugten Stellung der Gelenkschenkel zueinander ein Endpunkt des vorgegebenen Abschnitts der Außenkontur einen Endpunkt des vorgegebenen Abschnitts der Innenkontur berührt. Vorteilhafterweise kann damit ein fester Endpunkt der relativen Bewegung der Ausnehmung gegenüber dem Führungskörper definiert werden. Ferner kann ein Abbilden des natürlichen Bewegungsablaufs verbessert werden.

Gemäß einer Weiterbildung kann vorgesehen sein, dass in der vollständig gebeugten Stellung der Gelenkschenkel zueinander ein Teil der Außenkontur flächig einen Teil der Innenkontur kontaktiert. Der Teil der Außenkontur kann dabei eine äußere Seite des tropfenförmigen Führungskörpers, insbesondere die Außenseite des Führungskörpers, sein. Der Teil der Innenkontur kann ferner eine äußere Seite der zweiten Kammer der Ausnehmung sein. Das flächige Kontaktieren begünstigt eine Kraftübertragung zwischen Außenkontur und Innenkontur, bei der übertragene Kräfte auf eine größere Fläche verteilt werden können. So können hohe Flächenerpressungen vermieden werden. Im Hinblick auf ein gebeugtes Extremitätengelenk bedeutet dies, dass Kräfte in der gebeugten Stellung des Extremitätengelenks flächig übertragen werden können. Ferner kann eine exakt definierte Stellung der Gelenkanordnung in der vollständig gebeugten Stellung erreicht werden.

Berührt in einer vollständig gestreckten Stellung der Gelenkschenkel zueinander der Anfangspunkt des vorgegebenen Abschnitts der Außenkontur den Anfangspunkt des vorgegebenen Abschnitts der Innenkontur und berührt in einer vollständig gebeugten Stellung der Gelenkschenkel zueinander der Endpunkt des vorgegebenen Abschnitts der Außenkontur den Endpunkt des vorgegebenen Abschnitts der Innenkontur, so kann in vorteilhafter Weise erreicht werden, dass während dem gesamten Verschwenken der Gelenkschenkel zueinander der vorgegebene Abschnitt der Außenkontur auf dem vorgegebenen Abschnitt der Innenkontur zumindest phasenweise abrollt.

Gemäß einer Weiterbildung der Erfindung kann vorgesehen sein, dass der Führungskörper in der vollständig gebeugten Stellung der Gelenkschenkel zueinander im Wesentlichen in der zweiten Kammer, insbesondere in einem oberen, bevorzugt proximalen, Bereich der zweiten Kammer angeordnet ist.

Gemäß einer Ausgestaltung kann vorgesehen sein, dass der Führungskörper in der vollständig gebeugten Stellung derart in der zweiten Kammer angeordnet ist, dass er lediglich eine Bewegung in einer einzigen Richtung relativ zu der Ausnehmung vollziehen kann. In anderen Worten bedeutet dies, dass ein weiteres Beugen der Gelenkanordnung nicht möglich ist. Die Gelenkanordnung kann ausgehend von der gebeugten Stellung lediglich ein Verschwenken in Richtung einer gestreckten Stellung vollziehen. So kann in vorteilhafter Weise erreicht werden, dass die Bewegung entsprechend einer natürlichen maximalen Beugung des Extremitätengelenks oder einer gewünschten maximalen Beugung des Extremitätengelenks begrenzt bzw. abgrenzbar ist. Eine Schädigung des Extremitätengelenks durch Überbeugung kann vermieden werden.

Erfindungsgemäß kann vorgesehen sein, dass der vorgegebene Abschnitt der Innenkontur im Wesentlichen U-förmig und/oder schalenförmig ausgebildet ist.

Gemäß einem Aspekt der Erfindung kann vorgesehen sein, dass der vorgegebene Abschnitt der Außenkontur im Wesentlichen wellenförmig ausgebildet ist. Insbesondere kann vorgesehen sein, dass der vorgegebene Abschnitt der Außenkontur in einem ersten Teilabschnitt eine Wellenform umfasst und in einem zweiten Teilabschnitt verglichen mit dem ersten Teilabschnitt weniger wellenförmig, insbesondere vergleichsweise flach, verläuft.

Eine Weiterbildung der Erfindung sieht vor, dass zumindest einer der vorgegebenen Abschnitte eine Kontur aufweist, die im Wesentlichen eine Polbahn des Extremitätengelenks beschreibt.

Dabei kann gemäß einem Aspekt der Erfindung vorgesehen sein, dass der vorgegebene Abschnitt der Innenkontur im Wesentlichen eine Gangpolbahn des Extremitätengelenks beschreibt.

Außerdem kann erfindungsgemäß vorgesehen sein, dass der vorgegebene Abschnitt der Außenkontur im Wesentlichen eine Rastpolbahn des Extremitätengelenks beschreibt.

Eine Polbahn beschreibt Momentanpole einer ebenen Starrkörperbewegung. Ein Momentanpol ist ein Raumpunkt, an dem zu einem Zeitpunkt der Starrkörperbewegung keine Bewegung stattfindet. In anderen Worten kann die Starrkörperbewegung zu diesem Zeitpunkt als reine Rotation um diesen Raumpunkt angesehen werden. Die Momentanpole der Starrkörperbewegung bilden die Polbahn. In Bezug auf ein raumfestes Bezugssystem beschreiben die auftretenden Momentanpole eine sogenannte Rastpolbahn, wohingegen die Momentanpole in Bezug auf ein körperfestes Bezugssystem eine sogenannte Gangpolbahn beschreiben. Für die Gang- und Rastpolbahn einer Starrkörperbewegung ist charakteristisch, dass diese gleich lang sind und die Gangpolbahn ohne Gleiten auf der Rastpolbahn abrollt.

Die Erfinder haben erkannt, dass der komplexe Bewegungsablauf eines Extremitätengelenks in vorteilhafter Weise mittels Polbahnen erfasst werden kann. Außerdem haben die Erfinder erkannt, dass diese Polbahnen auf eine Gelenkanordnung für eine Orthese des Extremitätengelenks übertragen werden können. So kann mit der Gelenkanordnung mit Polbahnen in einfacher Weise ein natürlicher Bewegungsablauf abgebildet werden.

In Bezug auf die erläuterte erfindungsgemäße Gelenkanordnung bedeutet dies, dass der vorgegebene Abschnitt der Innenkontur der Ausnehmung auf dem vorgegebenen Abschnitt der Außenkontur des Führungskörpers abrollt und dabei die natürliche Bewegung des Kniegelenks abbildet. In anderen Worten rollt der vorgegebene Abschnitt der Innenkontur, der die Gangpolbahn des Extremitätengelenks beschreibt, auf dem vorgegebenen Abschnitt der Außenkontur ab, der die Rastpolbahn des Extremitätengelenks beschreibt. Ferner kann erreicht werden, dass eine Polbahn, insbesondere eine Gangpolbahn bzw. eine Rastpolbahn, individuell für einen Patienten berücksichtigt werden kann.

Gemäß einem Aspekt der Erfindung kann vorgesehen sein, dass der Anfangspunkt der Innenkontur einen Anfangspunkt der Gangpolbahn beschreibt.

Außerdem kann erfindungsgemäß vorgesehen sein, dass der Anfangspunkt der Au-βenkontur einen Anfangspunkt der Rastpolbahn beschreibt.

Ferner kann gemäß einem Aspekt der Erfindung des vorgesehen sein, dass der Endpunkt der Innenkontur einen Endpunkt der Gangpolbahn beschreibt.

Eine Weiterbildung der Erfindung sieht vor, dass der Endpunkt der Außenkontur einen Endpunkt der Rastpolbahn beschreibt.

Insgesamt kann durch die wie erläutert ausgestalteten Anfangs- bzw. Endpunkte erreicht werden, dass ein natürlicher Bewegungsablauf des Extremitätengelenks nachgebildet exakt und dennoch einfach werden kann.

Gemäß einer bevorzugten Ausführungsform der Erfindung kann vorgesehen sein, dass zwischen dem ersten und zweiten Kopfabschnitt ein reibungsminderndes Element, insbesondere eine Gleitscheibe, vorgesehen ist. Somit kann Reibung zwischen den Kopfabschnitten reduziert werden, wenn diese sich relativ zueinander bewegen und dabei miteinander kontaktieren. Insgesamt kann damit eine verbesserte Gelenkanordnung bereitgestellt werden, die leicht bewegbar ist.

Gemäß einem Aspekt der Erfindung kann vorgesehen sein, dass zumindest ein Verbindungsmittel, insbesondere ein Bolzen, eine Schraube oder ein Niet, und/oder ein formloser Hilfsstoff, bevorzugt ein Klebstoff, den Führungskörper an dem zweiten Kopfabschnitt befestigt. Damit kann erreicht werden, dass der Führungskörper fest an dem zweiten Kopfabschnitt angebracht ist. Ferner kann der Führungskörper unabhängig von dem zweiten Kopfabschnitt gefertigt werden. Außerdem kann erreicht werden, dass der Führungskörper einfach an dem zweiten Kopfabschnitt angebracht werden kann.

Gemäß einer alternativen Ausführungsform kann vorgesehen sein, dass der Führungskörper integral an dem zweiten Kopfabschnitt ausgebildet ist.

Gemäß einer Ausgestaltung der Erfindung kann vorgesehen sein, dass zumindest einer von dem ersten oder zweiten Gelenkschenkel einen dritten Kopfabschnitt umfasst. Der dritte Kopfabschnitt ist dabei dazu ausgebildet, mit dem jeweiligen anderen Kopfabschnitt des Gelenkschenkels zusammenzuwirken. Ferner kann der dritte Kopfabschnitt dazu ausgebildet sein, zusammen mit dem anderen Kopfabschnitt des Gelenkschenkels den Kopfabschnitt des anderen Gelenkschenkels zumindest an zwei Seiten zu umschließen. In vorteilhafter Weise kann damit eine einfache und robuste Gelenkanordnung erreicht werden.

Ferner kann erfindungsgemäß vorgesehen sein, dass einem der Gelenkschenkel zumindest ein Anschlagelement zugeordnet ist, das dazu eingerichtet ist, mit einem korrespondierenden Anschlagelement zusammenzuwirken, das dem anderen Gelenkschenkel zugeordnet ist, sodass das Verschwenken der Gelenkschenkel zueinander eingeschränkt oder einschränkbar ist. So kann beispielsweise erreicht werden, dass ein Verschwenken der Gelenkschenkel zueinander nur bis zu einem vorgegebenen bzw. vorgebbaren Ausmaß möglich ist. Auch ist es dadurch möglich, dass das Verschenken der Gelenkschenkel zueinander nur innerhalb eines vorgegebenen bzw. vorgebbaren Winkelbereichs möglich ist. Ferner kann dadurch erreich werden, dass das Ausmaß des Verschwenkens der Gelenkschenkel zueinander an einen Patienten angepasst werden kann. Dies kann beispielsweise durch unterschiedliche Therapien an Patienten oder Krankheitsbildern vorteilhaft sein.

Eine Weiterbildung der Erfindung sieht vor, dass das Extremitätengelenk ein Kniegelenk ist. Dabei kann insbesondere vorgesehen sein, dass das Kniegelenk ein menschliches Kniegelenk ist. Somit kann eine Gelenkanordnung bereitgestellt werden, die einem Kniegelenk angepasst ist.

Alternativ kann in einer Ausgestaltung vorgesehen sein, dass das Extremitätengelenk ein Ellenbogengelenk, ein Zehengelenk, ein Fingergelenk oder ein vergleichbares Gelenk eines menschlichen oder tierischen Körpers ist.

Eine Weiterbildung der Erfindung sieht vor, dass die Gelenkanordnung individuell für einen Patienten angepasst ist. Insbesondere kann vorgesehen sein, dass der vorgegebene Abschnitt der Innenkontur und/oder der vorgegebene Abschnitt der Außenkontur individuell für einen Patienten angepasst ist. Damit ist es in vorteilhafter Weise möglich, unterschiedliche natürliche Bewegungsabläufe von Patienten zu berücksichtigen. Insbesondere ist dies vorteilhaft, wenn eine Polbahn, insbesondere eine Gangpolbahn oder eine Rastpolbahn, dafür Patienten individuell ausfällt.

Ferner wird die eingangs bezeichnete und der Erfindung zugrunde liegende Aufgabe durch eine Orthese gelöst, wobei die Orthese zumindest eine Gelenkanordnung der vorangehenden beschriebenen Art umfasst.

Gemäß einer Weiterbildung der Erfindung kann vorgesehen sein, dass an der Orthese die zumindest eine Gelenkanordnung an einer lateralen oder einer medialen Seite des Extremitätengelenks angeordnet ist.

Gemäß einem Aspekt der Erfindung kann vorgesehen sein, dass an der Orthese eine erste Gelenkanordnung an einer lateralen Seite des Extremitätengelenks und eine zweite Gelenkanordnung an einer medialen Seite des Extremitätengelenks angeordnet ist. So kann erreicht werden, dass das Extremitätengelenk beiderseits gestützt wird.

Eine Weiterbildung der Erfindung sieht vor, dass die Orthese individuell für einen Patienten angepasst ist. Insbesondere kann vorgesehen sein, dass der vorgegebene Abschnitt der Innenkontur und/oder der vorgegebene Abschnitt der Außenkontur individuell für einen Patienten angepasst ist. Damit ist es in vorteilhafter Weise möglich, unterschiedliche natürliche Bewegungsabläufe von Patienten zu berücksichtigen. Insbesondere ist dies vorteilhaft, wenn eine Polbahn, insbesondere eine Gangpolbahn oder eine Rastpolbahn, dafür Patienten individuell ausfällt.

Gemäß einem Aspekt kann die erfindungsgemäße Orthese eine dem ersten Schenkelabschnitt zuordenbare Befestigungsvorrichtung und ferner eine dem zweiten Schenkelabschnitt zuordenbare Befestigungsvorrichtung umfassen, wobei die Befestigungsvorrichtungen dazu eingerichtet sind, an einer Extremität befestigt zu werden. Die Befestigungsvorrichtung kann ein Gurtsystem insbesondere mit einem Klettverschlusssystem umfassen. So kann erreicht werden, dass die Orthese einfach an einem Patienten angelegt werden kann. Ferner kann die Orthese einfach von dem Patienten wieder abgenommen werden. Außerdem kann die Orthese einfach an den Patienten angepasst werden.

Gemäß einer Ausgestaltung der erfindungsgemäßen Orthese kann vorgesehen sein, dass zumindest an einem Schenkelabschnitt eine einstellbare Verbindungsvorrichtung vorgesehen ist, die dazu eingerichtet ist, eine zugeordnete Befestigungsvorrichtung in unterschiedlichen Ausrichtungen gegenüber dem Schenkelabschnitt mit dem Schenkelabschnitt zu verbinden. Dies erleichtert es, die Orthese individuell an einen Patienten anzupassen.

Die Erfindung wird im Folgenden beispielhaft anhand der beiliegenden Figuren erläutert. Es stellen dar:
- Fig. 1: eine schematische Seitenansicht einer erfindungsgemäßen Gelenkanordnung in einer ersten Stellung;
- Fig. 2a: eine schematische Seitenansicht eines erfindungsgemäßen ersten Gelenkschenkels;
- Fig. 2b: eine schematische Seitenansicht eines erfindungsgemäßen zweiten Gelenkschenkels;
- Fig. 3: eine schematische Seitenansicht einer erfindungsgemäßen Gelenkanordnung in einer zweiten Stellung;
- Fig. 4: eine schematische Seitenansicht einer erfindungsgemäßen Gelenkanordnung in einer dritten Stellung;
- Fig. 5: eine schematische Seitenansicht einer erfindungsgemäßen Gelenkanordnung in einer vierten Stellung;
- Fig. 6: eine schematische Seitenansicht einer erfindungsgemäßen Gelenkanordnung in einer fünften Stellung;
- Fig. 7: eine schematische Explosionszeichnung einer erfindungsgemäßen Gelenkanordnung;
- Fig. 8: eine schematische Explosionszeichnung einer erfindungsgemäßen alternativen Gelenkanordnung;
- Fig. 9: eine schematische Explosionszeichnung einer erfindungsgemäßen alternativen Gelenkanordnung;
- Fig. 10: eine erste schematische Darstellung eines Bewegungsablaufs in einem Kniegelenk; und
- Fig. 11: eine zweite schematische Darstellung eines Bewegungsablaufs in einem Kniegelenk;

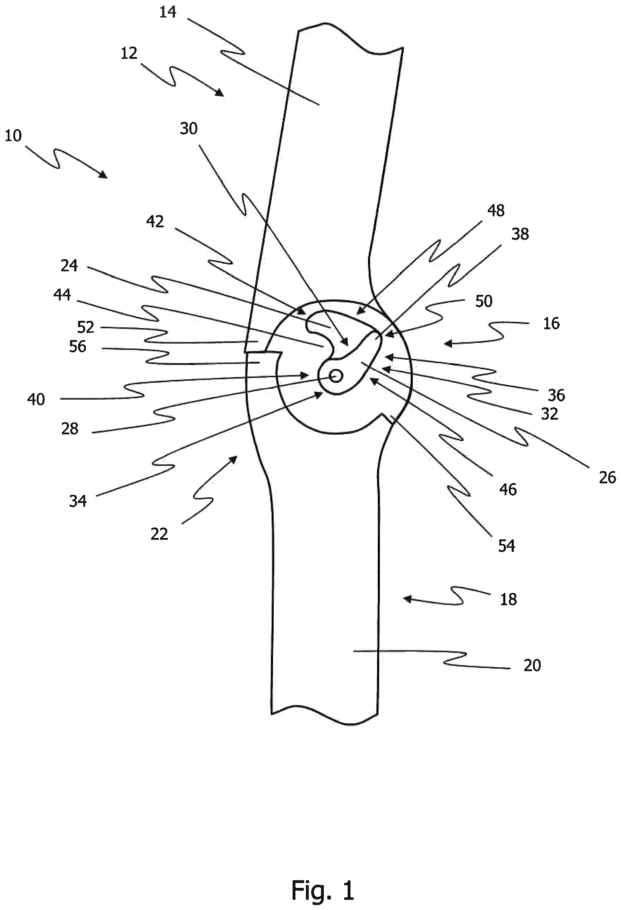

Figur 1 zeigt schematische Seitenansicht einer erfindungsgemäßen Gelenkanordnung 10 in einer ersten Stellung. In der ersten Stellung befindet sich die Gelenkanordnung 10 in einer vollständig gestreckten Stellung. Die Gelenkanordnung 10 umfasst einen ersten Gelenkschenkel 12 mit einem länglichen ersten Schenkelabschnitt 14 und einem ersten Kopfabschnitt 16. Ferner umfasst die Gelenkanordnung 10 einen zweiten Gelenkschenkel 18 mit einem länglichen zweiten Schenkelabschnitt 20 und einem zweiten Kopfabschnitt 22. Im Bereich des ersten und zweiten Kopfabschnitts 16, 22 überlappen sich der erste und zweite Gelenkschenkel 12, 18. Außerdem liegt der erste Kopfabschnitt 16 an dem zweiten Kopfabschnitt 22 an. Der längliche erste Schenkelabschnitt 14 und der längliche zweite Schenkelabschnitt 20 sind mit einer geschwungenen Linie jeweils verkürzt dargestellt. Dies soll verdeutlichen, dass die Länge des länglichen ersten und zweiten Schenkelabschnitts 14, 20 einsatzabhängig und/oder Patientenabhängig gewählt werden kann. Ferner können nicht dargestellte Komponenten vorgesehen sein, um die Gelenkanordnung 10 in eine Orthese zu integrieren.

An dem ersten Kopfabschnitt 16 ist eine Ausnehmung 24 ausgebildet. Ferner ist an dem zweiten Kopfabschnitt 22 ein Führungskörper 26 ausgebildet. Der Führungskörper 26 ist in der Ausnehmung 24 angeordnet. Ferner ist der Führungskörper 26 in der Ausnehmung 24 bewegbar angeordnet bzw. die Ausnehmung 24 um den Führungskörper 26 bewegbar angeordnet. Dadurch, dass der erste Kopfabschnitt 16 an dem zweiten Kopfabschnitt 22 anliegt und beide Kopfabschnitte 16, 22 mit im Wesentlichen ebenen Seitenflächen ausgebildet sind, sind der erste Gelenkschenkel 12 und der zweite Gelenkschenkel 18 in einer Schwenkebene zueinander verschwenkbar. Der erste Gelenkschenkel 12 ist somit gelenkig mit dem zweiten Gelenkschenkel 18 verbunden. Ob sich der Führungskörper 26 in der Ausnehmung 24 bewegt oder ob sich die Ausnehmung 24 um den Führungskörper 26 bewegt, ist davon abhängig, welcher der Gelenkschenkel 12, 18 fixiert oder verschwenkt wird. Grundsätzlich ist es auch möglich, beide Gelenkschenkel 12, 18 zu verschwenken. Wird der erste Gelenkschenkel 12 fixiert und der zweite Gelenkschenkel 18 verschwenkt, so bewegt sich der Führungskörper 26 in der Ausnehmung 24. Wird der zweite Gelenkschenkel 18 fixiert und der erste Gelenkschenkel 12 verschwenkt, so bewegt sich die Ausnehmung 24 um den Führungskörper 26. Der Einfachheit halber wird von einer relativen Bewegung zwischen Führungskörper 26 und Ausnehmung 24 gesprochen. Damit sollen beide Bewegungen umfasst sein.

Der Führungskörper 26 ist mittels eines Verbindungsmittels 28 an dem zweiten Kopfabschnitt 22 befestigt. Der Führungskörper 26 umfasst eine Außenkontur, wobei die Außenkontur tropfenförmig und gekrümmt ausgebildet ist. So umfasst der Führungskörper 26 eine Innenseite 30 und eine Außenseite 32. Die Innenseite 30 ist bezogen auf die gekrümmte Form des Führungskörpers 26 innenliegend angeordnet. Die Außenseite 32 ist bezogen auf die gekrümmte Form des Führungskörpers 26 außenliegend angeordnet. Ferner umfasst der Führungskörper einen verdickten Kopfbereich 34 und einen sich verjüngenden Fortsatz 36. Der verdickten Kopfbereich 34 ist rund ausgebildet und umfasst zumindest teilweise eine kreisrunde Form. Der Fortsatz 36 umfasst ein Fortsatzende 38, wobei das Fortsatzende 38 rund ausgebildet ist. Die Ausnehmung 24 umfasst eine Innenkontur, wobei die Innenkontur herzförmig ausgebildet. Die Ausnehmung 24 umfasst eine erste Kammer 40 und eine zweite Kammer 42. In der gezeigten Darstellung ist die erste Kammer 40 unterhalb der zweiten Kammer 42 angeordnet. In Bezug auf einen Patienten kann die erste Kammer 40 distal und die zweite Kammer 42 proximal angeordnet sein. Ferner ist zwischen der ersten Kammer 40 und der zweiten Kammer 42 ein Vorsprung 44 ausgebildet. Der Vorsprung 44 ist mit runder Form ausgebildet. Die erste Kammer 40 weist eine Kontur auf, die derart ausgebildet ist, dass sie den verdickten Kopfbereich 34 des Führungskörpers 26 aufnehmen kann. Vorliegend ist die erste Kammer 40 rund ausgebildet und umfasst zumindest teilweise eine kreisrunde Form. Die erste Kammer 40 umfasst ferner einen seitlichen ersten Kammerabschnitt 46. Ferner umfasst die zweite Kammer 42 einen seitlichen zweiten Kammerabschnitt 48. Der seitliche erste Kammerabschnitt 46 und der seitliche zweite Kammerabschnitt 48 bilden die Seiten der Herzform. An einer Unterseite der Herzform und zwischen dem seitlichen ersten Kammerabschnitt 46 und dem seitlichen zweiten Kammerabschnitt 48 ist ein Herzformboden 50 ausgebildet. Der Herzformboden 50 ist rund ausgebildet. Ferner verbindet der Herzformboden 50 den seitlichen ersten Kammerabschnitt 46 und den seitlichen zweiten Kammerabschnitt 48. Der Herzformboden 50 ist derart ausgebildet, dass er das Fortsatzende 38 des Führungskörpers 26 zumindest teilweise umschließen kann.

Der Führungskörper 26 ist in der gezeigten Darstellung derart in der Ausnehmung 24 angeordnet, dass er in der ersten Kammer 40 angeordnet ist. Der verdickten Kopfbereich 34 ist dabei in der kreisrunden Form der ersten Kammer 40 teilweise eingefasst bzw. von dieser umschlossen. Ferner liegt die Außenseite 32 des Führungskörpers 26 flächig an dem seitlichen ersten Kammerabschnitt 48 an. Die Form der Außenseite 32 stimmt mit der Form des seitlichen ersten Kammerabschnitts 48 überein. Das Fortsatzende 38 des Führungskörpers 26 ist in dem Herzformboden 50 angeordnet. Lediglich die Innenseite 30 des Führungskörpers 26 kontaktiert nicht bzw. fast nicht und insbesondere nur in einem Kontaktpunkt mit der Ausnehmung 24. Es ist erkennbar, dass die Formen von Führungskörper 26 und Ausnehmung 24 aufeinander abgestimmt sind. Schließlich ist der Führungskörper 26 jedoch derart frei in der Ausnehmung 24 eingefasst, dass eine genau definierte, zwangsgeführte relative Bewegung zwischen Führungskörper 26 und Ausnehmung 24 ermöglicht wird.

An dem ersten Gelenkschenkel 12 sind ferner ein erstes Anschlagelement 52 und ein zweites Anschlagelement 54 ausgebildet. Genauer gesagt sind das erste Anschlagelement 52 und das zweite Anschlagelement 54 im Bereich des ersten Kopfabschnitts 16 angeordnet. Das erste Anschlagelement 52 ist nahe zu dem Vorsprung 44 ausgebildet. Das zweite Anschlagelement 54 ist nahe zu dem seitlichen ersten Kammerabschnitt 46 angeordnet.

Ferner ist an dem zweiten Gelenkschenkel 18 ein drittes Anschlagelement 56 ausgebildet. Das dritte Anschlagelement 56 ist im Bereich des zweiten Kopfabschnitts 22 angeordnet. Das dritte Anschlagelement 56 ist außerdem nahe dem verdickten Kopfbereich 34 des Führungskörpers 26 angeordnet.

Die Anschlagelemente 52, 54, 56 sind als Stufen mit einer geraden Fläche ausgebildet. Das erste Anschlagelement 52 und das dritte Anschlagelement 56 liegen in der gezeigten Darstellung flächig aneinander an.

Der erste Gelenkschenkel 12 und der zweite Gelenkschenkel 18 sind in der gezeigten Darstellung durchsichtig dargestellt, sodass die Form des ersten Kopfabschnitts 16 erkennbar ist, wenn auch der erste Kopfabschnitt 16 teilweise von dem zweiten Kopfabschnitt 22 überlappt ist.

Der erste Gelenkschenkel 12 ist gegenüber dem zweiten Gelenkschenkel 18 unterschiedlich ausgerichtet. In der gezeigten Darstellung ist der erste Gelenkschenkel 12 gegenüber dem zweiten Gelenkschenkel 18 um einen Winkel von etwa 10° verschwenkt. Einen bestimmten Winkel vorzusehen kann vorteilhaft sein, um eine natürliche gestreckte Stellung eines Extremitätengelenks abzubilden. Ferner kann dies für eine Integration der Gelenkanordnung in einer Orthese vorteilhaft sein. Alternativ kann der Winkel auch 0° betragen.

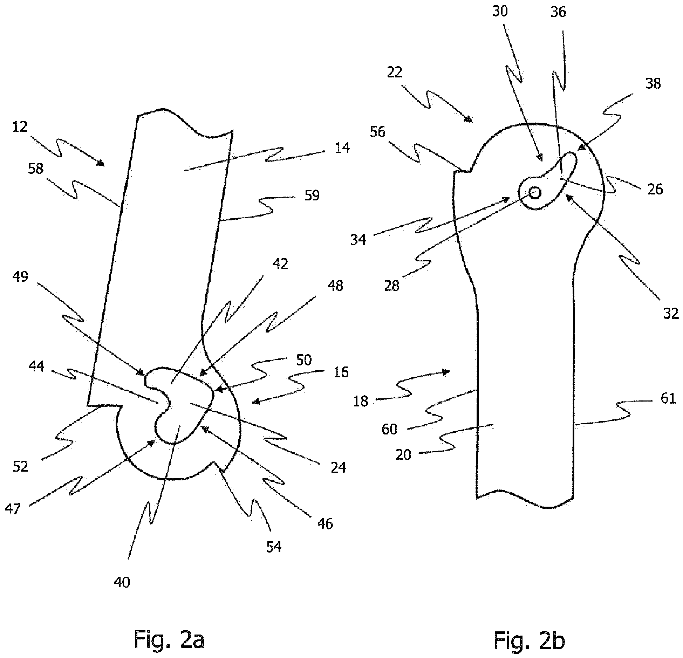

Figur 2a zeigt eine schematische Seitenansicht des erfindungsgemäßen ersten Gelenkschenkels 12. An dem ersten Kopfabschnitt 16 ist die Ausnehmung 24 ausgebildet. Die Ausnehmung 24 umfasst die erste Kammer 40 und die zweite Kammer 42. Zwischen der ersten Kammer 40 und der zweiten Kammer 42 ist der Vorsprung 44 ausgebildet. Die erste Kammer 40 umfasst den seitlichen ersten Kammerabschnitt 46, während die zweite Kammer 42 den seitlichen zweiten Kammerabschnitt 48 umfasst. Ferner umfasst die erste Kammer 40 einen rund ausgebildeten ersten Kammerkopf 47. Die zweite Kammer 42 umfasst einen rund ausgebildeten zweiten Kammerkopf 49. Der zweite Kammerkopf 49 ist kleiner ausgebildet als der erste Kammerkopf 47. Zwischen dem seitlichen ersten Kammerabschnitt 46 und den seitlichen zweiten Kammerabschnitt 48 ist der Herzformboden 50 ausgebildet. Der zweite Kammerkopf 49 ist insbesondere deshalb kleiner ausgebildet als der erste Kammerkopf 47, um den Fortsatz 36 und das Fortsatzende 38 besser zu umschließen. So kann eine definierte gebeugte Stellung erreicht werden.

Im Bereich des ersten Kopfabschnitts 16 sind das erste Anschlagelement 52 und das zweite Anschlagelement 54 ausgebildet. Der erste Kopfabschnitt 16 ist zwischen dem ersten Anschlagelement 52 und dem zweiten Anschlagelement 54 kreisrund ausgebildet. Der längliche erste Schenkelabschnitt 14 umfasst zwei parallele Seiten 58, 59. Ferner ist der längliche erste Schenkelabschnitt 14 gegenüber dem ersten Kopfabschnitt 16 versetzt angeordnet. Genauer gesagt ist eine mittig angeordnete Längsachse des länglichen ersten Schenkelabschnitts 14 gegenüber einem Zentrum des ersten Kopfabschnitts 16 versetzt angeordnet.

Figur 2b zeigt eine schematische Seitenansicht des erfindungsgemäßen zweiten Gelenkschenkels 18. An dem zweiten Kopfabschnitt 22 ist der Führungskörper 26 ausgebildet. Der Führungskörper 26 umfasst die Innenseite 30 und die Außenseite 32. Ferner umfasst der Führungskörper den verdickten Kopfbereich 34 und den sich verjüngenden Fortsatz 36. An dem Fortsatz 36 ist das Fortsatzende 38 ausgebildet.

Der Führungskörper 26 ist mittels des Verbindungsmittels 28 an dem zweiten Kopfabschnitt 22 fixiert.

Im Bereich des zweiten Kopfabschnitts 22 ist das dritte Anschlagelement 56 ausgebildet. Der zweite Kopfabschnitt 22 ist abgesehen von dem dritten Anschlagelement 56 im Wesentlichen rund und teilweise kreisrund ausgebildet. Der längliche zweite Schenkelabschnitt 20 ist im Wesentlichen mittig gegenüber dem zweiten Kopfabschnitt 22 angeordnet. Ferner umfasst der längliche zweite Schenkelabschnitt zueinander parallele Seiten 60, 61.

Beide Gelenkschenkel 12, 18 sind in Draufsicht dargestellt. Daher sei angemerkt, dass die Gelenkschenkel 12, 18 jeweils eine konstante Dicke aufweisen.

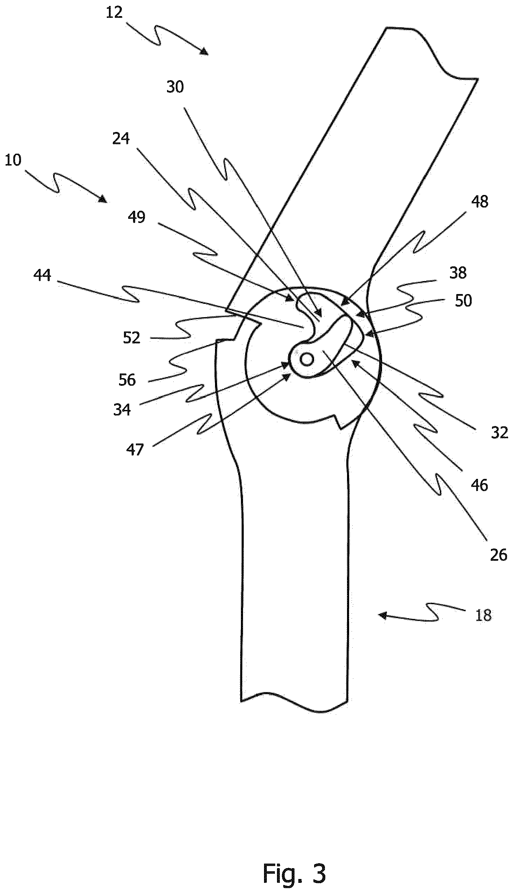

Figur 3 zeigt eine schematische Seitenansicht der erfindungsgemäßen Gelenkanordnung 10 in einer zweiten Stellung. Gegenüber der Darstellung in Figur 1 ist erkennbar, dass der erste Gelenkschenkel 12 gegenüber dem zweiten Gelenkschenkel 18 verschwenkt ist. Genauer gesagt ist der erste Gelenkschenkel 12 gegenüber dem zweiten Gelenkschenkel 18 um einen Winkel von etwa 30° verschwenkt dargestellt. Der zweite Gelenkschenkel 18 ist während dem Verschwenken fixiert, sodass sich seine Position nicht geändert hat. Das erste Anschlagelement 52 ist nun beabstandet zu dem dritten Anschlagelement 56.

Es ist erkennbar, dass eine relative Bewegung zwischen Führungskörper 26 und Ausnehmung 24 stattgefunden hat. Genauer gesagt hat sich der erste Kammerkopf 47 der Ausnehmung 24 um den verdickten Kopfbereich 34 des Führungskörpers 26 gedreht. Dabei hat der erste Kammerkopf 47 relativ zu den verdickten Kopfbereich 34 eine Gleitbewegung ausgeführt.

Ferner liegt die Außenseite 32 des Führungskörpers 26 zumindest nicht mehr vollständig an dem seitlichen ersten Kammerabschnitt 46 an. Vielmehr schließt die Außenseite 32 mit dem seitlichen ersten Kammerabschnitt 46 einen Winkel ein.

Das Fortsatzende 38 kontaktiert nicht mehr den Herzformboden 50. Vielmehr kontaktiert das Fortsatzende 38 den seitlichen zweiten Kammerabschnitt 48 in einem mittleren Bereich. In anderen Worten hat sich das Fortsatzende 38 von dem Herzformboden 50 entlang des seitlichen zweiten Kammerabschnitts 48 bewegt. Das Fortsatzende 38 hat relativ zu dem seitlichen zweiten Kammerabschnitt 48 eine Translationsbewegung vollzogen. Außerdem hat sich das Fortsatzende 38 dem zweiten Kammerkopf 49 angenähert.

Weiterhin ist erkennbar, dass der Vorsprung 44 nahe der Innenseite 30 des Führungskörpers 26 angeordnet ist und diese jedoch gerade noch nicht kontaktiert.

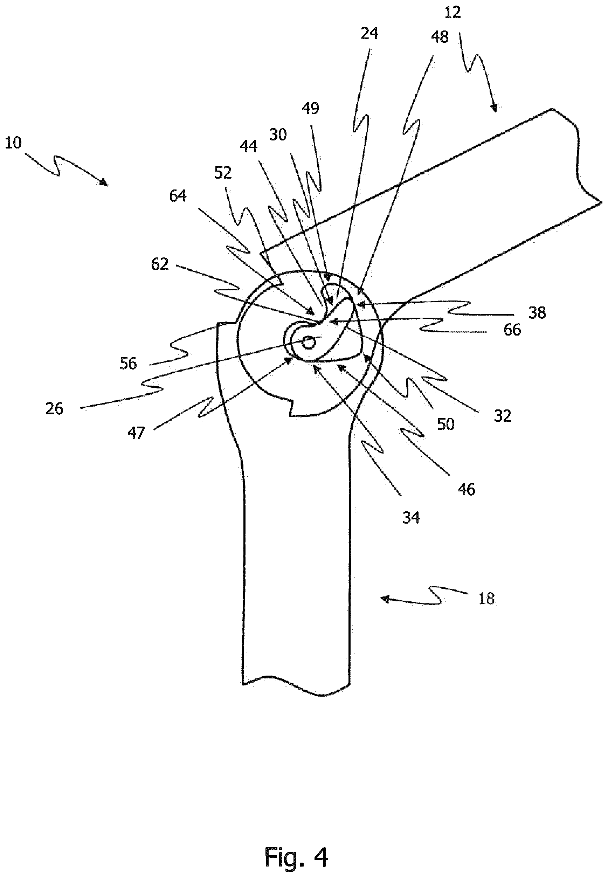

Figur 4 zeigt eine schematische Seitenansicht der erfindungsgemäßen Gelenkanordnung 10 in einer dritten Stellung. Gegenüber Figur 3 ist erkennbar, dass der erste Gelenkschenkel 12 gegenüber dem zweiten Gelenkschenkel 18 weiter verschwenkt ist. Genauer gesagt ist der erste Gelenkschenkel 12 gegenüber dem zweiten Gelenkschenkel 18 um einen Winkel von etwa 64° verschwenkt dargestellt. Wie bereits zuvor ist der zweite Gelenkschenkel 18 während dem Verschwenken fixiert, sodass sich seine Position nicht geändert hat. Das erste Anschlagelement 52 ist noch weiter entfernt von dem dritten Anschlagelement 56.

Es ist erkennbar, dass wiederum eine relative Bewegung zwischen Führungskörper 26 und Ausnehmung 24 stattgefunden hat. Der erste Kammerkopf 47 ist nun beabstandet zu dem verdickten Kopfbereich 34 des Führungskörpers 26 angeordnet. Der verdickte Kopfbereich 34 des Führungskörpers 26 kontaktiert den seitlichen ersten Kammerabschnitt 46. Gegenüber Figur 3 ist erkennbar, dass sich der verdickte Kopfbereich 34 entlang des seitlichen ersten Kammerabschnitts 46 in Richtung Herzformboden 50 bewegt hat.

Weiterhin ist die Außenseite 32 des Führungskörpers 26 weiter entfernt von dem seitlichen ersten Kammerabschnitt 46. Gegenüber Figur 3 hat sich der Winkel vergrößert, den die Außenseite 32 mit dem seitlichen ersten Kammerabschnitt 46 einschließt.

Weiterhin kontaktiert das Fortsatzende 38 den seitlichen zweiten Kammerabschnitt 48. Dabei hat sich das Fortsatzende 38 entlang des seitlichen zweiten Kammerabschnitts 48 bewegt. In anderen Worten hat das Fortsatzende 38 relativ zu dem seitlichen zweiten Kammerabschnitt 48 eine Translationsbewegung vollzogen. Das Fortsatzende 38 hat sich dem zweiten Kammerkopf 49 weiter angenähert.

Ferner ist erkennbar, dass der Vorsprung 44 die Innenseite 30 des Führungskörpers 26 kontaktiert. Genauer gesagt kontaktiert der Vorsprung 44 die Innenseite 30 des Führungskörpers 26 in einem ersten Kontaktpunkt 62. An dem ersten Kontaktpunkt 62 rollt ein vorgegebener Abschnitt der Innenkontur 64 der Ausnehmung 24 an einem vorgegebenen Abschnitt der Außenkontur 66 des Führungskörpers 26 ab. Der vorgegebene Abschnitt der Innenkontur 64 der Ausnehmung 24 vollzieht eine Rollbewegung auf dem vorgegebenen Abschnitt der Außenkontur 66 des Führungskörpers 26.

Der vorgegebene Abschnitt der Innenkontur 64 und der vorgegebene Abschnitt der Außenkontur 66 sind einer Polbahn des menschlichen Kniegelenks nachempfunden. Genauer gesagt ist der vorgegebene Abschnitt der Innenkontur 64 an dem Vorsprung 44 ausgebildet. Ferner beschreibt der vorgegebene Abschnitt der Innenkontur 64 eine Gangpolbahn des menschlichen Kniegelenks. Der vorgegebene Abschnitt der Außenkontur 66 ist an der Innenseite 30 des Führungskörpers 26 ausgebildet. Ferner beschreibt der vorgegebene Abschnitt der Innenkontur 66 eine Rastpolbahn des menschlichen Kniegelenks.

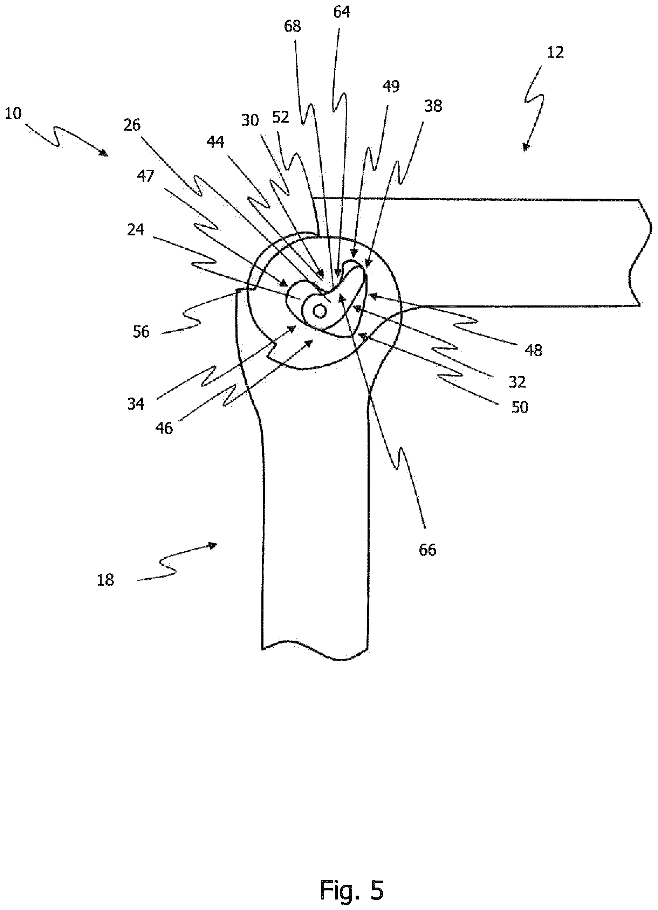

Figur 5 zeigt eine schematische Seitenansicht der erfindungsgemäßen Gelenkanordnung 10 in einer vierten Stellung. In der vierten Stellung ist der erste Gelenkschenkel 12 im Wesentlichen senkrecht gegenüber dem zweiten Gelenkschenkel 18 angeordnet. Gegenüber Figur 4 ist erkennbar, dass der erste Gelenkschenkel 12 gegenüber dem zweiten Gelenkschenkel 18 weiter verschwenkt ist. Wie bereits zuvor ist der zweite Gelenkschenkel 18 während dem Verschenken fixiert, sodass sich seine Position nicht geändert hat. Das erste Anschlagelement 52 ist noch weiter entfernt von dem dritten Anschlagelement 56.

Es ist erkennbar, dass wiederum eine relative Bewegung zwischen Führungskörper 26 und Ausnehmung 24 stattgefunden hat. Der erste Kammerkopf 47 ist beabstandet zu dem verdickten Kopfbereich 34 des Führungskörpers 26. Gegenüber Figur 4 ist der erste Kammerkopf 47 weiter beabstandet gegenüber dem verdickten Kopfbereich 34. Der verdickte Kopfbereich 34 des Führungskörpers 26 kontaktiert den seitlichen ersten Kammerabschnitt 46. Gegenüber Figur 4 ist erkennbar, dass sich der verdickte Kopfbereich 34 entlang des seitlichen ersten Kammerabschnitts 46 in Richtung Herzformboden 50 bewegt hat.

Die Außenseite 32 des Führungskörpers 26 ist wiederum weiter entfernt von dem seitlichen ersten Kammerabschnitt 46. Gegenüber Figur 4 hat sich der Winkel vergrößert, den die Außenseite 32 mit dem seitlichen ersten Kammerabschnitt 46 einschließt. Vielmehr hat sich die Außenseite 32 dem seitlichen zweiten Kammerabschnitt 48 angenähert.

Weiterhin kontaktiert das Fortsatzende 38 den seitlichen zweiten Kammerabschnitt 48. Dabei hat sich das Fortsatzende 38 entlang des seitlichen zweiten Kammerabschnitts 48 bewegt. In anderen Worten hat das Fortsatzende 38 relativ zu dem seitlichen zweiten Kammerabschnitt 48 eine Translationsbewegung vollzogen. Das Fortsatzende 38 hat sich dem zweiten Kammerkopf 49 weiter angenähert. Das Fortsatzende 38 ist jedoch noch nicht vollständig in dem zweiten Kammerkopf 49 angeordnet.

Außerdem ist erkennbar, dass der Vorsprung 44 weiterhin die Innenseite 30 des Führungskörpers 26 kontaktiert. Genauer gesagt kontaktiert der Vorsprung 44 die Innenseite 30 des Führungskörpers 26 in einem zweiten Kontaktpunkt 68. An dem zweiten Kontaktpunkt 68 rollt der vorgegebene Abschnitt der Innenkontur 64 an dem vorgegebenen Abschnitt der Außenkontur 66 ab. Der vorgegebene Abschnitt der Innenkontur 64 der Ausnehmung 24 vollzieht eine Rollbewegung auf dem vorgegebenen Abschnitt der Außenkontur 66 des Führungskörpers 26. Gegenüber Figur 4 ist erkennbar, dass der zweite Kontaktpunkt 68 gegenüber dem ersten Kontaktpunkt 62 weiter entfernt von dem verdickten Kopfbereich 34 und näher zu dem Fortsatzende 38 des Führungskörpers 26 angeordnet ist.

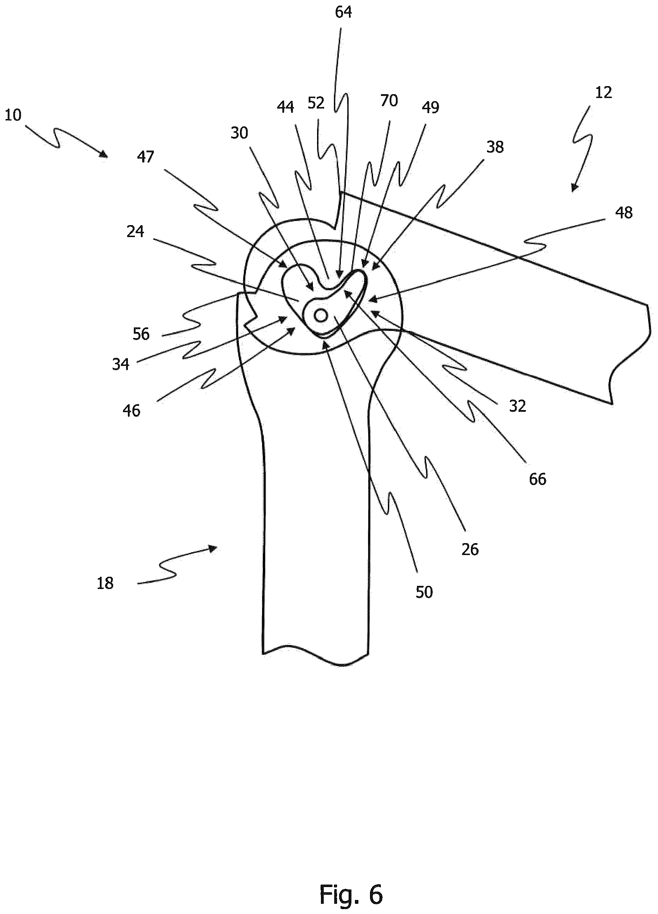

Figur 6 zeigt eine schematische Seitenansicht der erfindungsgemäßen Gelenkanordnung 10 in einer fünften Stellung. Gegenüber Figur 5 ist erkennbar, dass der erste Gelenkschenkel 12 gegenüber dem zweiten Gelenkschenkel 18 weiter verschwenkt ist. Genauer gesagt ist der erste Gelenkschenkel 12 gegenüber dem zweiten Gelenkschenkel 18 um einen Winkel von etwa 110° verschwenkt dargestellt. Dieser Winkel kann in Abhängigkeit von der Außenkontur des Führungskörpers 26 und der Innenkontur der Ausnehmung 24 angepasst werden. Wie bereits zuvor ist der zweite Gelenkschenkel 18 während dem Verschwenken fixiert, sodass sich seine Position nicht geändert hat. Das erste Anschlagelement 52 ist noch weiter entfernt von dem dritten Anschlagelement 56.

Es ist erkennbar, dass wiederum eine relative Bewegung zwischen Führungskörper 26 und Ausnehmung 24 stattgefunden hat. Der erste Kammerkopf 47 ist beabstandet zu dem verdickten Kopfbereich 34 des Führungskörpers 26. Gegenüber Figur 5 ist der erste Kammerkopf 47 weiter beabstandet gegenüber dem verdickten Kopfbereich 34. Der verdickte Kopfbereich 34 des Führungskörpers 26 kontaktiert den seitlichen ersten Kammerabschnitt 46 und den seitlichen zweiten Kammerabschnitt 48. Gegenüber Figur 5 ist erkennbar, dass sich der verdickte Kopfbereich 34 entlang des seitlichen ersten Kammerabschnitts 46 bewegt hat. Dabei hat sich der verdickte Kopfbereich 34 dem Herzformboden 50 angenähert und ist im Wesentlichen bei dem Herzformboden 50 angeordnet.

Die Außenseite 32 des Führungskörpers 26 ist dem seitlichen ersten Kammerabschnitt 46 abgewandt. Vielmehr berührt die Außenseite 22 den seitlichen zweiten Kammerabschnitt 48.

Das Fortsatzende 38 kontaktiert den zweiten Kammerkopf 49 und ist in diesem angeordnet. Es ist erkennbar, dass sich das Fortsatzende 38 entlang des seitlichen zweiten Kammerabschnitts 48 bewegt hat. In anderen Worten hat das Fortsatzende 38 relativ zu dem seitlichen zweiten Kammerabschnitt 48 eine Translationsbewegung vollzogen.

Ferner ist erkennbar, dass der Vorsprung 44 weiterhin die Innenseite 30 des Führungskörpers 26 kontaktiert. Genauer gesagt kontaktiert der Vorsprung 44 die Innenseite 30 des Führungskörpers 26 in einem dritten Kontaktpunkt 70. An dem dritten Kontaktpunkt 70 rollt der vorgegebene Abschnitt der Innenkontur 64 an den vorgegebenen Abschnitt der Außenkontur 66 ab. Der vorgegebene Abschnitt der Innenkontur 64 der Ausnehmung 24 vollzieht eine Rollbewegung auf dem vorgegebenen Abschnitt der Außenkontur 66 des Führungskörpers 26. Gegenüber Figur 5 ist erkennbar, dass der dritte Kontaktpunkt 70 gegenüber dem ersten Kontaktpunkt 62 und dem zweiten Kontaktpunkt 68 weiter entfernt von dem verdickten Kopfbereich 34 entlang des Vorsprungs 44 und näher zu dem Fortsatzende 38 des Führungskörpers 26 angeordnet ist.

Der Führungskörper 26 ist nun im Wesentlichen in dem seitlichen zweiten Kammerabschnitt 48 angeordnet. Ferner ist der Führungskörper 26 derart in dem seitlichen zweiten Kammerabschnitt 48 angeordnet, dass keine weitere Bewegung in Richtung des seitlichen zweiten Kammerabschnitts 48 bzw. in Richtung des zweiten Kammerkopfes 49 möglich ist. Vielmehr kann lediglich eine Bewegung vollzogen werden, mit der sich der Führungskörper 26 wieder zurück in Richtung des seitlichen ersten Kammerabschnitts 46 bzw. in Richtung des ersten Kammerkopfes 47 bewegt.

Der erste Schenkelabschnitt 12 ist gegenüber dem zweiten Schenkelabschnitt 18 vollständig verschwenkt.

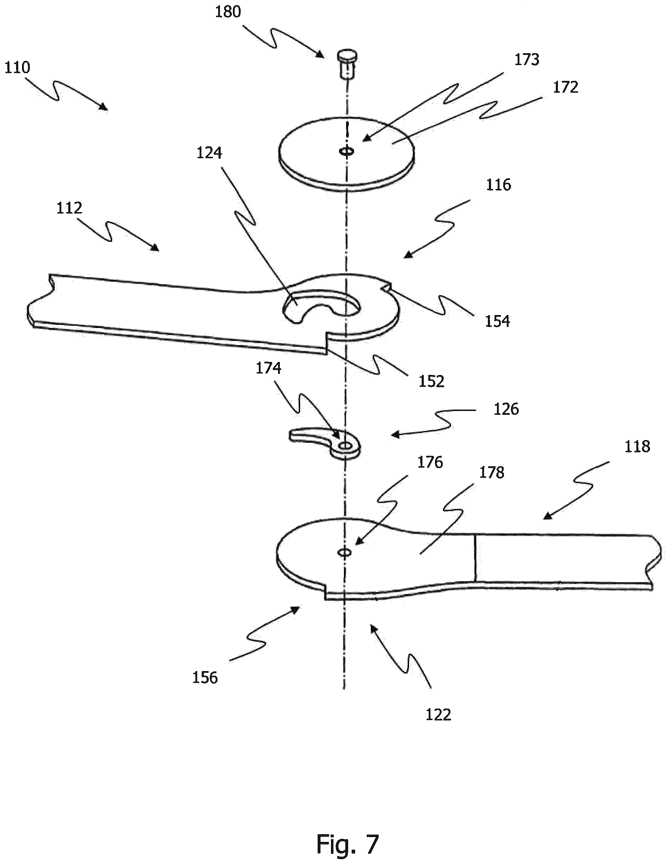

Figur 7 zeigt eine schematische Explosionszeichnung einer erfindungsgemäßen Gelenkanordnung 110. Die Gelenkanordnung 110 ist der bisherigen Gelenkanordnung 10 ähnlich. Im Folgenden wird daher insbesondere auf Unterschiede eingegangen oder Merkmale werden genauer erläutert, die nun verbessert erkennbar sind. Gegenüber der Gelenkanordnung 10 umfasst die Gelenkanordnung 110 eine erste Gelenkscheibe 172 mit einer Gelenkscheibenöffnung 173. Die erste Gelenkscheibe 172 kann eine Gleitscheibe sein. Die erste Gelenkscheibe 172 ist kreisrund ausgebildet. Ferner weist die erste Gelenkscheibe 172 eine konstante Dicke auf.

An einem ersten Gelenkschenkel 112 ist im Bereich eines ersten Kopfabschnitts 116 eine Ausnehmung 124 angeordnet. Die Ausnehmung 124 ist herzförmig ausgebildet. An dem ersten Gelenkschenkel sind im Bereich des ersten Kopfabschnitts 116 ein erstes Anschlagelement 152 und ein zweites Anschlagelement 154 ausgebildet. Der erste Gelenkschenkel 112 weist eine konstante Dicke auf. Eine Oberseite des ersten Gelenkschenkels 112 ist parallel zu einer Unterseite des ersten Gelenkschenkels ausgebildet.

Ferner ist ein Führungskörper 126 dargestellt, wobei der Führungskörper 126 tropfenförmig ausgebildet ist und eine Führungskörperöffnung 174 umfasst.

An einem zweiten Gelenkschenkel 118 ist im Bereich eines zweiten Kopfabschnitts 122 eine Öffnung 176 ausgebildet. Ferner ist an dem zweiten Gelenkschenkel 118 im Bereich des zweiten Kopfabschnitts 122 ein drittes Anschlagelement 156 ausgebildet. Außerdem ist an dem zweiten Gelenkschenkel 118 im Bereich des zweiten Kopfabschnitts 122 eine aufbereitete Oberfläche 178 vorgesehen. Der zweite Gelenkschenkel 118 weist eine konstante Dicke auf. Eine Oberseite des zweiten Gelenkschenkels 118 ist parallel zu einer Unterseite des ersten Gelenkschenkels ausgebildet.

Ferner ist ein Bolzen 180 dargestellt. Der Bolzen 180 ist dazu ausgebildet, die erste Gelenkscheibe 172, den ersten Gelenkschenkel 112, den Führungskörper 126 und den zweiten Gelenkschenkel 118 miteinander zu verbinden. In einem verbundenen Zustand erstreckt sich der Bolzen 180 durch die Gelenkscheibenöffnung 173 und die Führungskörperöffnung 174 bis in die Öffnung 176 des zweiten Gelenkschenkels 118. In dem verbundenen Zustand kontaktiert die erste Gelenkscheibe 172 eine Oberseite des ersten Gelenkschenkels 112 im Bereich des ersten Kopfabschnitts 116. Der Führungskörper 126 ist in der Ausnehmung 124 angeordnet. Ferner ist der Führungskörper 126 auf dem zweiten Gelenkschenkel 118 fest angeordnet. Neben dem Bolzen 180 kann zusätzlich oder alternativ ein Verbindungsmittel wie ein Klebstoff vorgesehen sein, das den Führungskörper 126 an dem zweiten Gelenkschenkel 118 positions- und drehfest anordnet.

Das erste und zweite Anschlagelement 152, 154 sind dazu ausgebildet, mit dem zweiten Gelenkschenkel 118 bzw. einer dem zweiten Gelenkschenkel 118 zugeordneten Komponente zusammenzuwirken. Insbesondere sind das erste bzw. zweite Anschlagelement 152, 154 dazu eingerichtet, jeweils eine korrespondierende und dem zweiten Gelenkschenkel 118 zugeordnete Anschlagfläche zu kontaktieren. Ferner ist das dritte Anschlagelement 156 dazu ausgebildet, mit dem ersten Gelenkschenkel 112 bzw. einer dem ersten Gelenkschenkel 112 zugeordneten Komponente zusammenzuwirken. Insbesondere ist das dritte Anschlagelement 156 dazu eingerichtet, eine korrespondierende und dem ersten Gelenkschenkel 112 zugeordnete Anschlagfläche zu kontaktieren. In Figur 7 sind diese Komponenten bzw. Anschlagflächen nicht näher dargestellt. Derartige Komponenten können weitere Anschlagelemente, Steckverbindungen, Feststellschrauben, Feststellklemmen, Anschlagscheiben, Anschlagkomponenten und/oder andere geeignete Komponenten sein. Das erste Anschlagelement 152 und das dritte Anschlagelement 156 können derart ausgebildet sein, dass sie in einer gestreckten Stellung miteinander kontaktieren. Insbesondere sind die Komponenten dazu eingerichtet, eine Begrenzung der Beugung bzw. Streckung vorzunehmen. Dadurch kann gezielt eine therapeutisch gewünschte Beugungs- bzw. Streckungsstellung der Gelenkanordnung 110 eingestellt werden. Ist eine Begrenzung der Beugung und/oder Streckung nicht notwendig, so können die Anschlagelemente 152, 154, 156 und/oder weitere nicht dargestellte Komponenten teilweise oder ganz entfallen.

Die aufbereitete Oberfläche 178 ist derart ausgebildet, dass sie eine geringe Oberflächenreibung aufweist. Dies ist vorteilhaft, um eine möglichst geringe Reibung zwischen dem ersten Kopfabschnitt 116 und dem zweiten Kopfabschnitt 122 zu erreichen, wenn der erste Gelenkschenkel 112 gegenüber dem zweiten Gelenkschenkel 118 verschwenkt wird. Ferner kann auch die erste Gelenkscheibe 172 zumindest an ihrer Unterseite eine derartig aufbereitete Oberfläche aufweisen.

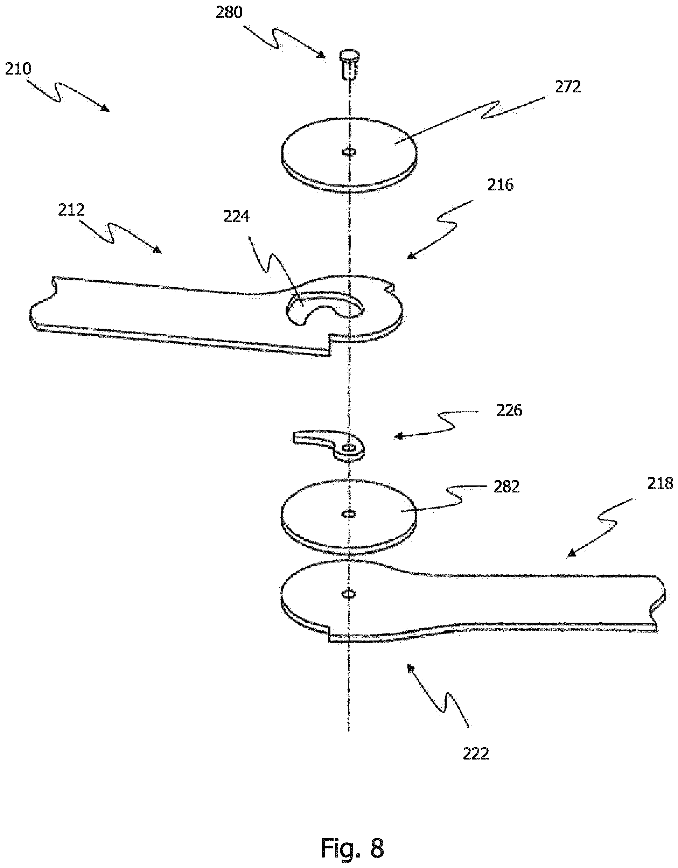

Figur 8 zeigt eine schematische Explosionszeichnung einer erfindungsgemäßen alternativen Gelenkanordnung 210. Die alternative Gelenkanordnung 210 ist ähnlich zu der Gelenkanordnung 110 ausgebildet und umfasst so einen Bolzen 280, eine erste Gelenkscheibe 272, einen ersten Gelenkschenkel 212 mit einem ersten Kopfabschnitt 216 und einer Ausnehmung 224, einen Führungskörper 226 und einen zweiten Gelenkschenkel 218 mit einem zweiten Kopfabschnitt 222. Die erste Gelenkscheibe 272 kann eine Gleitscheibe sein. Anders als die Gelenkanordnung 110 umfasst die alternative Gelenkanordnung 210 zusätzlich eine zweite Gelenkscheibe 282. Die zweite Gelenkscheibe 282 kann eine Gleitscheibe sein. Die zweite Gelenkscheibe 282 ist zwischen dem Führungskörper 226 und dem zweiten Gelenkschenkel 218 angeordnet. Die zweite Gelenkscheibe 282 ist dazu eingerichtet, mit dem Führungskörper 226 und dem ersten Gelenkschenkel 212 einerseits und mit dem zweiten Gelenkschenkel 218 andererseits zu kontaktieren. Die zweite Gelenkscheibe 218 ist an einer Oberseite reibungsvermindert. Dies ist vorteilhaft, um eine möglichst geringe Reibung zwischen dem ersten Kopfabschnitt 216 und dem zweiten Kopfabschnitt 222 zu erreichen, wenn der erste Gelenkschenkel 212 gegenüber dem zweiten Gelenkschenkel 218 verschwenkt wird. Es versteht sich, dass die zweite Gelenkscheibe 282 fest und insbesondere drehfest mit dem zweiten Gelenkschenkel 212 verbunden ist. Außerdem ist die zweite Gelenkscheibe 282 fest und insbesondere drehfest mit dem Führungskörper 226 verbunden.

Alternativ zu der Ausgestaltung mit einer zweiten Gelenkscheibe 282 ist es auch möglich, zumindest eine Unterseite des ersten Gelenkschenkels 212, insbesondere im Bereich der ersten Kopfabschnitts 222, reibungsvermindert auszubilden.

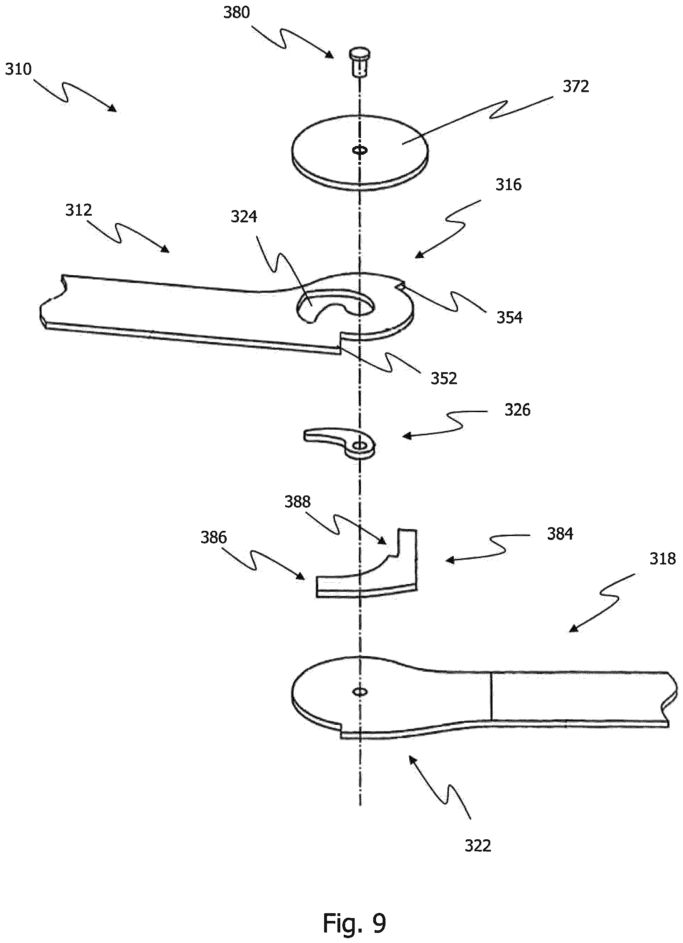

Figur 9 zeigt eine schematische Explosionszeichnung einer erfindungsgemäßen alternativen Gelenkanordnung 310. Die alternative Gelenkanordnung 310 ist ähnlich zu der Gelenkanordnung 110 ausgebildet und umfasst so einen Bolzen 380, eine erste Gelenkscheibe 372, einen ersten Gelenkschenkel 312 mit einem ersten Kopfabschnitt 316 und einer Ausnehmung 324, einen Führungskörper 326 und einen zweiten Gelenkschenkel 318 mit einem zweiten Kopfabschnitt 322. Die erste Gelenkscheibe 372 kann eine Gleitscheibe sein. Anders als die Gelenkanordnung 110 umfasst die alternative Gelenkanordnung 310 zusätzlich eine Anschlagvorrichtung 384. Die Anschlagvorrichtung 384 umfasst eine erste Anschlagfläche 386 und eine zweite Anschlagfläche 388. Die erste Anschlagfläche 386 ist dazu ausgebildet, mit einem ersten Anschlagelement 352 des ersten Gelenkschenkels 312 zusammenzuwirken. Die zweite Anschlagfläche 388 ist dazu ausgebildet, mit einem zweiten Anschlagelement 354 des ersten Gelenkschenkels 312 zusammenzuwirken. Die Anschlagvorrichtung 384 ist an ihrer Innenseite derart geformt, dass sie mit einem Bereich an dem ersten Kopfabschnitt 316 zwischen der ersten Anschlagfläche 386 und der zweiten Anschlagfläche 388 flächig kontaktiert. Die Anschlagvorrichtung 384 weist eine konstante Dicke auf.

Abhängig von der Position der ersten Anschlagfläche 386 bzw. der Position der zweiten Anschlagfläche 388 an der Anschlagvorrichtung 384 ist es möglich, das Ausmaß des Verschwenkens zwischen erstem Gelenkschenkel 312 und zweitem Gelenkschenkel 318 durch Kontaktieren mit dem ersten Anschlagelement 352 bzw. dem zweiten Anschlagelement 354 zu begrenzen. So kann eine vollständig gestreckte Stellung zwischen erstem Gelenkschenkel 312 und zweitem Gelenkschenkel 318 bedarfsabhängig angepasst werden. Auch kann eine vollständig gebeugte Stellung zwischen erstem Gelenkschenkel 312 und zweitem Gelenkschenkel 318 bedarfsabhängig angepasst werden. In der vollständig gestreckten Stellung kontaktiert die erste Anschlagfläche 386 das erste Anschlagelement 352. In der vollständig gebeugten Stellung kontaktiert die zweite Anschlagfläche 388 das zweite Anschlagelement 354. Die Anschlagvorrichtung 384 kann alternativ auch als eine Steckverbindung, eine Feststellschraube, eine Feststellklemme oder als eine anders ausgebildete Anschlagvorrichtung ausgebildet sein.

In einem montierten Zustand kontaktiert die erste Gelenkscheibe 372 den ersten Gelenkschenkel 312 und den Führungskörper 326. Ferner kontaktieren der erste Gelenkschenkel 312 und der Führungskörper 326 jeweils an der anderen Seite mit dem zweiten Gelenkschenkel 318. Der Bereich an dem ersten Kopfabschnitt 316 zwischen der ersten Anschlagfläche 386 und der zweiten Anschlagfläche 388 kontaktiert zumindest teilweise die Anschlagvorrichtung 384 an ihrer Innenseite.

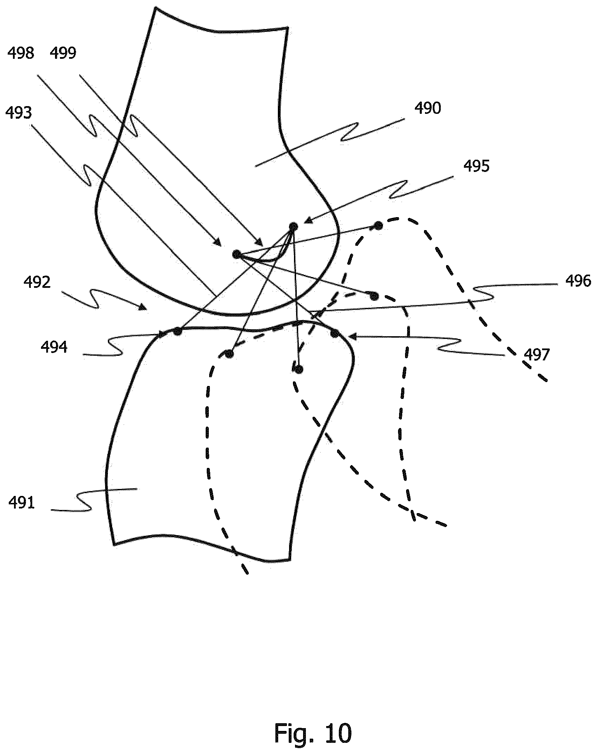

Figur 10 zeigt eine erste schematische Darstellung eines Bewegungsablaufs in einem Kniegelenk. Vereinfacht sind Femur 490 und Tibia 491 in einer ersten Stellung dargestellt. Zudem ist die Tibia 491 strichliert in einer zweiten Stellung und strichliert in einer dritten Stellung dargestellt. Zwischen Femur 490 und Tibia 491 ist ein Gelenkspalt 492 ausgebildet. Femur 490 und Tibia 491 sind in der ersten Stellung in einer gestreckten Stellung bzw. in Extension des Kniegelenks dargestellt, während die zweite und dritte Stellung der Tibia 491 gebeugte Stellungen bzw. Stellungen zwischen Extension und Flexion des Kniegelenks darstellen.

Ferner ist ein erstes Band 493 dargestellt, insbesondere ein erstes Kreuzband des Kniegelenks, das mit der Tibia 491 in einem ersten Verbindungpunkt 494 und mit der Femur 490 in einem zweiten Verbindungspunkt 495 verbunden ist. Ferner ist ein zweites Band 496 dargestellt, insbesondere ein zweites Kreuzband des Kniegelenks, das mit der Tibia 491 in einem dritten Verbindungspunkt 497 und mit der Femur 490 in einem vierten Verbindungpunkt 498 verbunden ist.

Außerdem ist exemplarisch eine Polbahn in Form einer Gangpolbahn 499 dargestellt. Die Gangpolbahn 499 beschreibt Momentanpole der relativen Bewegung zwischen Femur 490 und Tibia 491, wobei die Femur 490 fixiert ist und die Tibia 491 relativ zu der Femur 490 verschwenkt wird.

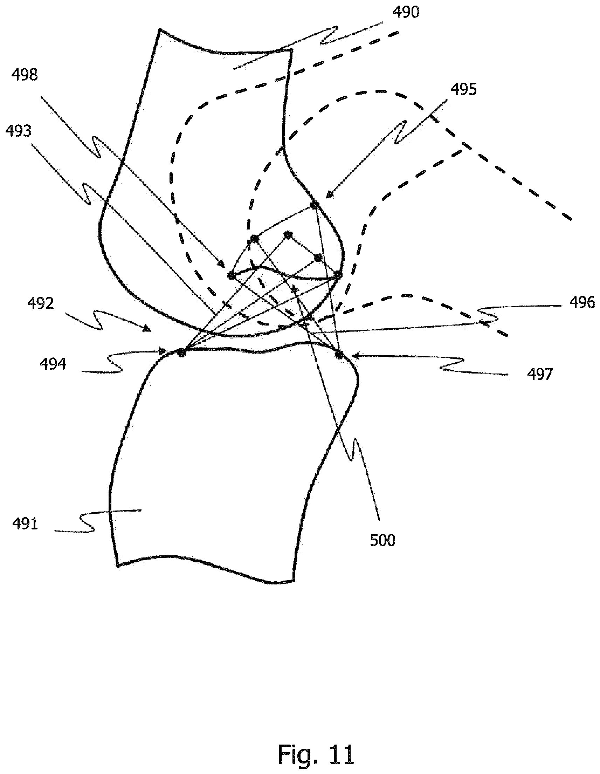

Figur 11 zeigt eine zweite schematische Darstellung eines Bewegungsablaufs in einem Kniegelenk. Wie in Figur 10 sind Femur 490 und Tibia 491 in der ersten Stellung dargestellt. Zudem ist die Femur 490 strichliert in einer vierten Stellung und strichliert in einer fünften Stellung dargestellt. Zwischen Femur 490 und Tibia 491 ist der Gelenkspalt 492 ausgebildet.

Ferner ist das erste Band 493 dargestellt, insbesondere ein erstes Kreuzband des Kniegelenks, das mit der Tibia 491 in dem ersten Verbindungpunkt 494 und mit der Femur 490 in dem zweiten Verbindungspunkt 495 verbunden ist. Ferner ist das zweite Band 496 dargestellt, insbesondere ein zweites Kreuzband des Kniegelenks, das mit der Tibia 491 in dem dritten Verbindungspunkt 497 und mit der Femur 490 in dem vierten Verbindungpunkt 498 verbunden ist.

Außerdem ist exemplarisch eine Polbahn in Form einer Rastpolbahn 500 dargestellt. Die Rastpolbahn 500 beschreibt Momentanpole der relativen Bewegung zwischen Femur 490 und Tibia 491, wobei die Tibia 491 fixiert ist und die Femur 490 relativ zu der Tibia 491 verschwenkt wird.

In der Zusammenschau von Figuren 10 und 11 kann festgestellt werden, dass die Gangpolbahn 499 auf der Rastpolbahn 500 abrollt bzw. umgekehrt die Rastpolbahn 500 auf der Gangpolbahn 499 abrollt, abhängig davon ob Femur 490 oder Tibia 491 fixiert werden. Ferner ist ersichtlich, dass die Bänder 493, 496 zusammen mit den Verbindungspunkten 494, 495, 497, 498 eine natürliche Bewegung vollziehen. Durch Abbilden der Polbahnen in der Gelenkanordnung 10, 110, 210, 310 kann erreicht werden, dass eine Belastung der Bänder reduziert und ein natürlicher Bewegungsablauf erzielt wird. Insbesondere ist erkennbar, dass der vorgegebene Abschnitt der Innenkontur der Ausnehmung 24, 124, 224, 324 die Gangpolbahn 500 beschreibt. Die Form des vorgegebenen Abschnitts der Innenkontur der Ausnehmung 24, 124, 224, 324 entspricht im Wesentlichen der Gangpolbahn 499. Außerdem ist ersichtlich, dass der vorgegebene Abschnitt der Außenkontur des Führungskörpers 26, 126, 226, 326 die Rastpolbahn 500 beschreibt. Die Form des vorgegebenen Abschnitts der Außenkontur des Führungskörpers 26, 126, 226, 326 entspricht im Wesentlichen der Rastpolbahn 500. Abhängig davon, wie die Rastpolbahn 500 bzw. die Gangpolbahn 499 bei einem Patienten ausfallen, kann der vorgegebene Abschnitt der Außenkontur oder der vorgegebene Abschnitt der Innenkontur angepasst sein und patientenabhängig ausfallen.

## Patentansprüche

1. Gelenkanordnung (10; 110; 210; 310) für eine Orthese eines Extremitätengelenks, umfassend
einen ersten Gelenkschenkel (12; 112; 212; 312) mit einem länglichen ersten Schenkelabschnitt (14) und einem ersten Kopfabschnitt (16; 116; 216; 316); und
einen zweiten Gelenkschenkel (18; 118; 218; 318) mit einem länglichen zweiten Schenkelabschnitt (20) und einem zweiten Kopfabschnitt (22; 122; 222; 322);
wobei sich der erste Gelenkschenkel (12; 112; 212; 312) und der zweite Gelenkschenkel (18; 118; 218; 318) im Bereich des ersten bzw. zweiten Kopfabschnitts (16; 116; 216; 316; 22; 122; 222; 322) zumindest teilweise flächig überlappenden und ferner derart gelenkig miteinander verbunden sind, dass die Gelenkschenkel (12; 112; 212; 312; 18; 118; 218; 318) in einer Schwenkebene zueinander verschwenkbar sind; und
wobei ferner in dem ersten Kopfabschnitt (16; 116; 216; 316) eine Ausnehmung (24; 124; 224; 324) und an dem zweiten Kopfabschnitt (22; 122; 222; 322) ein Führungskörper (26; 126; 226; 326) vorgesehen ist, wobei der Führungskörper (26; 126; 226; 326) in der Ausnehmung (24; 124; 224; 324) und relativ zu dieser bewegbar angeordnet ist;
**dadurch gekennzeichnet, dass** die Ausnehmung (24; 124; 224; 324) eine Innenkontur und der Führungskörper (26; 126; 226; 326) eine Außenkontur aufweisen, die derart ausgebildet sind, dass während einem Verschwenken der Gelenkschenkel (12; 112; 212; 312; 18; 118; 218; 318) zueinander die Ausnehmung (24; 124; 224; 324) relativ zu dem Führungskörper eine Rotations- und Translationsbewegung ausführt, bei der sie um den Führungskörper (26; 126; 226; 326) zwangsgeführt ist und zumindest phasenweise ein vorgegebener Abschnitt der Innenkontur (64) auf einem vorgegebenen Abschnitt der Außenkontur (66) abrollt.

2. Gelenkanordnung (10; 110; 210; 310) nach Anspruch 1, **dadurch gekennzeichnet, dass** der Führungskörper (26; 126; 226; 326) tropfenförmig ausgebildet ist, wobei insbesondere der Führungskörper (26; 126; 226; 326) einen verdickten Kopfbereich (34) und einen sich verjüngenden Fortsatz (36) umfasst.

3. Gelenkanordnung (10; 110; 210; 310) nach einem der vorangehenden Ansprüche, **dadurch gekennzeichnet, dass** der Führungskörper (26; 126; 226; 326) bogenförmig und/oder harmonisch gekrümmt ausgebildet ist und insbesondere eine Innenseite (30) des Führungskörpers (26; 126; 226; 326) im Wesentlichen den vorgegebenen Abschnitt der Außenkontur (66) umfasst.

4. Gelenkanordnung (10; 110; 210; 310) nach einem der vorangehenden Ansprüche, **dadurch gekennzeichnet, dass** die Ausnehmung (24; 124; 224; 324) herzförmig ausgebildet ist.

5. Gelenkanordnung (10; 110; 210; 310) nach Anspruch 5, **dadurch gekennzeichnet, dass** die Ausnehmung (24; 124; 224; 324) eine erste, insbesondere distale, Kammer (40) und eine zweite, bevorzugt proximale, Kammer (42) umfasst, wobei ein Vorsprung (44) zwischen den Kammern (40, 42) ausgebildet ist, wobei insbesondere der Vorsprung (44) im Wesentlichen den vorgegebenen Abschnitt der Innenkontur (64) umfasst.

6. Gelenkanordnung (10; 110; 210; 310) nach einem der vorangehenden Ansprüche, **dadurch gekennzeichnet, dass** in einer vollständig gestreckten Stellung der Gelenkschenkel (12; 112; 212; 312; 18; 118; 218; 318) zueinander ein Anfangspunkt des vorgegebenen Abschnitts der Außenkontur (66) einen Anfangspunkt des vorgegebenen Abschnitts der Innenkontur (64) berührt und insbesondere ein Teil der Außenkontur flächig einen Teil der Innenkontur kontaktiert.

7. Gelenkanordnung (10; 110; 210; 310) nach Ansprüchen 5 und 6, **dadurch gekennzeichnet, dass** der Führungskörper (26; 126; 226; 326) in der vollständig gestreckten Stellung der Gelenkschenkel (12; 112; 212; 312; 18; 118; 218; 318) zueinander im Wesentlichen in der ersten Kammer (40), insbesondere in einem unteren, bevorzugt distalen, Bereich der ersten Kammer (40), angeordnet ist.

8. Gelenkanordnung (10; 110; 210; 310) nach einem der vorangehenden Ansprüche, **dadurch gekennzeichnet, dass** in einer vollständig gebeugten Stellung der Gelenkschenkel (12; 112; 212; 312; 18; 118; 218; 318) zueinander ein Endpunkt des vorgegebenen Abschnitts der Außenkontur (66) einen Endpunkt des vorgegebenen Abschnitts der Innenkontur (64) berührt und insbesondere ein Teil der Außenkontur flächig einen Teil der Innenkontur kontaktiert, wobei insbesondere der Führungskörper (26; 126; 226; 326) in der vollständig gebeugten Stellung der Gelenkschenkel (12; 112; 212; 312; 18; 118; 218; 318) zueinander im Wesentlichen in der zweiten Kammer (42), insbesondere in einem oberen, bevorzugt proximalen, Bereich der zweiten Kammer (42) angeordnet ist.

9. Gelenkanordnung (10; 110; 210; 310) nach einem der vorangehenden Ansprüche, **dadurch gekennzeichnet, dass** zumindest einer der vorgegebenen Abschnitte eine Kontur aufweist, die im Wesentlichen eine Polbahn des Extremitätengelenks beschreibt, wobei insbesondere der vorgegebene Abschnitt der Innenkontur (64) im Wesentlichen eine Gangpolbahn (499) des Extremitätengelenks beschreibt.

10. Gelenkanordnung (10; 110; 210; 310) nach Anspruch 9, **dadurch gekennzeichnet, dass** der vorgegebene Abschnitt der Außenkontur (66) im Wesentlichen eine Rastpolbahn (500) des Extremitätengelenks beschreibt.

11. Gelenkanordnung (10; 110; 210; 310) nach einem der vorangehenden Ansprüche, **dadurch gekennzeichnet, dass** zwischen dem ersten Kopfabschnitt (16; 116; 216; 316) und dem zweiten Kopfabschnitt (22; 122; 222; 322) ein reibungsminderndes Element, insbesondere eine Gleitscheibe, vorgesehen ist.

12. Gelenkanordnung (10; 110; 210; 310) nach einem der vorangehenden Ansprüche, **dadurch gekennzeichnet, dass** zumindest ein Verbindungsmittel (28), insbesondere ein Bolzen (180; 280; 380), eine Schraube oder ein Niet, und/oder ein formloser Hilfsstoff, bevorzugt ein Klebstoff, den Führungskörper (26; 126; 226; 326) an dem zweiten Kopfabschnitt (22; 122; 222; 322) befestigt.

13. Gelenkanordnung (10; 110; 210; 310) nach einem der vorangehenden Ansprüche, **dadurch gekennzeichnet, dass** einem der Gelenkschenkel (12; 112; 212; 312; 18; 118; 218; 318) zumindest ein Anschlagelement (52, 54, 56) zugeordnet ist, das dazu eingerichtet ist, mit einem korrespondierenden Anschlagelement (52, 54, 56) zusammenzuwirken, das dem anderen Gelenkschenkel (12; 112; 212; 312; 18; 118; 218; 318) zugeordnet ist, sodass das Verschwenken der Gelenkschenkel (12; 112; 212; 312; 18; 118; 218; 318) zueinander eingeschränkt oder einschränkbar ist.

14. Gelenkanordnung (10; 110; 210; 310) nach einem der vorangehenden Ansprüche, **dadurch gekennzeichnet, dass** das Extremitätengelenk ein Kniegelenk, besonders bevorzugt eines menschlichen Kniegelenks ist.

15. Orthese, umfassend zumindest eine Gelenkanordnung (10; 110; 210; 310) nach einem der Ansprüche 1 bis 14, wobei insbesondere die zumindest eine Gelenkanordnung (10; 110; 210; 310) an einer lateralen oder einer medialen Seite des Extremitätengelenks angeordnet ist, wobei bei der Orthese vorzugsweise vorgesehen ist, dass diese umfasst eine dem ersten Schenkelabschnitt (14) zuordenbare Befestigungsvorrichtung und ferner eine dem zweiten Schenkelabschnitt (20) zuordenbare Befestigungsvorrichtung, wobei die Befestigungsvorrichtungen dazu eingerichtet sind, an einer Extremität befestigt zu werden, wobei bei der Orthese ferner oder alternativ optional vorgesehen ist, dass zumindest an einem Schenkelabschnitt (14, 20) eine einstellbare Verbindungsvorrichtung vorgesehen ist, die dazu eingerichtet ist, eine zugeordnete Befestigungsvorrichtung in unterschiedlichen Ausrichtungen gegenüber dem Schenkelabschnitt (14, 20) mit dem Schenkelabschnitt (14, 20) zu verbinden.

## Claims

1. An articulated arrangement (10; 110; 210; 310) for an orthosis of an extremity joint, comprising
a first articulated leg (12; 112; 212; 312) having an elongated first leg portion (14) and a first head portion (16; 116; 216; 316); and
a second articulated leg (18; 118; 218; 318) having an elongated second leg portion (20) and a second head portion (22; 122; 222; 322);
wherein the first articulated leg (12; 112; 212; 312) and the second articulated leg (18; 118; 218; 318) at least partially overlap in the area of the first and second head portion (16; 116; 216; 316; 22; 122; 222; 322), respectively, and are further connected to each other in an articulated manner so that the articulated legs (12; 112; 212; 312; 18; 118; 218; 318) may be pivoted relative to each other in a pivot plane; and
wherein a recess (24; 124; 224; 324) is further provided in the first head portion (16; 116; 216; 316) and a guide body (26; 126; 226; 326) is provided in the second head portion (22; 122; 222; 322), wherein the guide body (26; 126; 226; 326) is arranged in and movable relative to the recess (24; 124; 224; 324);
**characterized in that** the recess (24; 124; 224; 324) has an inner contour and the guide body (26; 126; 226; 326) has an outer contour, which are configured to cause the recess (24; 124; 224; 324), while the articulated legs (12; 112; 212; 312; 18; 118; 218; 318) pivot relative to one another, to perform a rotational and translational movement relative to the guide body during which it is positively guided around the guide body (26; 126; 226; 326) and, at least in certain phases, a predetermined portion of the inner contour (64) rolls on a predetermined portion of the outer contour (66).

2. The articulated arrangement (10; 110; 210; 310) according to claim 1, **characterized in that** the guide body (26; 126; 226; 326) is drop-shaped, wherein the guide body (26; 126; 226; 326) in particular comprises a thickened head portion (34) and a tapering extension (36).

3. The articulated arrangement (10; 110; 210; 310) according to one of the preceding claims, **characterized in that** the guide body (26; 126; 226; 326) is arcshaped and/or harmonically curved and that, in particular, an inner side (30) of the guide body (26; 126; 226; 326) essentially comprises the predetermined portion of the outer contour (66).

4. The articulated arrangement (10; 110; 210; 310) according to one of the preceding claims, **characterized in that** the recess (24; 124; 224; 324) is heartshaped.

5. The articulated arrangement (10; 110; 210; 310) according to claim 5, **characterized in that** the recess (24; 124; 224; 324) comprises a first, particularly distal, chamber (40) and a second, preferably proximal, chamber (42), wherein a protrusion (44) is formed between the chambers (40, 42), wherein in particular the protrusion (44) essentially comprises the predetermined portion of the inner contour (64).

6. The articulated arrangement (10; 110; 210; 310) according to one of the preceding claims, **characterized in that**, with the articulated legs (12; 112; 212; 312; 18; 118; 218; 318) fully extended relative to one another, an initial point of the predetermined portion of the outer contour (66) touches an initial point of the predetermined portion of the inner contour (64) and, in particular, a part of the outer contour touches a part of the inner contour in a planar fashion.

7. The articulated arrangement (10; 110; 210; 310) according to claims 5 and 6, **characterized in that**, with the articulated legs (12; 112; 212; 312; 18; 118; 218; 318) fully extended relative to one another, the guide body (26; 126; 226; 326) is essentially arranged in the first chamber (40), in particular in a lower, preferably distal, area of the first chamber (40).

8. The articulated arrangement (10; 110; 210; 310) according to one of the preceding claims, **characterized in that**, with the articulated legs (12; 112; 212; 312; 18; 118; 218; 318) fully bent relative to one another, an end point of the predetermined portion of the outer contour (66) touches an end point of the predetermined portion of the inner contour (64) and, in particular, a part of the outer contour touches a part of the inner contour in a planar fashion, wherein the guide body (26; 126; 226; 326), in particular, with the articulated legs (12; 112; 212; 312; 18; 118; 218; 318) fully bent relative to one another, is essentially arranged in the second chamber (42), in particular in an upper, preferably proximal, area of the second chamber (42).

9. The articulated arrangement (10; 110; 210; 310) according to any one of the preceding claims, **characterized in that** at least one of the predetermined portions has a contour which essentially describes a centrode of the extremity joint, wherein the predetermined portion of the inner contour (64), in particular, essentially describes a moving centrode (499) of the extremity joint.

10. The articulated arrangement (10; 110; 210; 310) according to claim 9, **characterized in that** the predetermined portion of the outer contour (66) essentially describes a fixed centrode (500) of the extremity joint.

11. The articulated arrangement (10; 110; 210; 310) according to one of the preceding claims, **characterized in that** a friction-reducing element, in particular a sliding disk, is provided between the first head portion (16; 116; 216; 316) and the second head portion (22; 122; 222; 322).

12. The articulated arrangement (10; 110; 210; 310) according to one of the preceding claims, **characterized in that** at least one connecting means (28), in particular a bolt (180; 280; 380), a screw or a rivet, and/or an amorphous auxiliary material, preferably an adhesive, fasten the guide body (26; 126; 226; 326) to the second head portion (22; 122; 222; 322).

13. The articulated arrangement (10; 110; 210; 310) according to one of the preceding claims, **characterized in that** at least one stop element (52, 54, 56) is associated with one of the articulated legs (12; 112; 212; 312; 18; 118; 218; 318), wherein the stop element is configured to interact with a corresponding stop element (52, 54, 56) associated with the other articulated leg (12; 112; 212; 312; 18; 118; 218; 318) so that pivoting the articulated legs (12; 112; 212; 312; 18; 118; 218; 318) relative to one another is or may be restricted.

14. The articulated arrangement (10; 110; 210; 310) according to one of the preceding claims, **characterized in that** the extremity joint is a knee joint, highly preferably a human knee joint.

15. An orthosis comprising at least one articulated arrangement (10; 110; 210; 310) according to one of claims 1 to 14, wherein in particular the at least one articulated arrangement (10; 110; 210; 310) is arranged on a lateral or a medial side of the extremity joint, wherein the orthosis is preferably configured to comprise a fastening device associable with the first leg portion (14) and a fastening device associable with the second leg portion (20), the fastening devices being configured to be fastened to an extremity, wherein the orthosis is further or as an alternative optionally configured to have on at least one leg portion (14, 20) at least one adjustable connecting device configured to connect an associated fastening device to the leg portion (14, 20) in different orientations relative to the leg portion (14, 20).

## Revendications

1. Ensemble articulé (10 ; 110 ; 210 ; 310) pour une orthèse d'une articulation d'extrémité, comprenant
une première branche d'articulation (12 ; 112 ; 212 ; 312) ayant une première section de branche allongée (14) et une première section de tête (16 ; 116 ; 216 ; 316) ; et
une deuxième branche d'articulation (18 ; 118 ; 218 ; 318) ayant une deuxième section de branche allongée (20) et une deuxième section de tête (22 ; 122 ; 222 ; 322) ;
dans lequel la première branche d'articulation (12 ; 112 ; 212 ; 312) et la deuxième branche d'articulation (18 ; 118 ; 218 ; 318) sont respectivement reliées dans la zone de la première et de la deuxième section de tête (16 ; 116 ; 216 ; 316 ; 22 ; 122 ; 222 ; 322) de manière à se chevaucher en surface au moins partiellement et en outre de manière articulée de telle sorte que les branches d'articulation (12 ; 112 ; 212 ; 312 ; 18 ; 118 ; 218 ; 318) peuvent pivoter l'une par rapport à l'autre dans un plan de pivotement ; et
dans lequel, en outre, un évidement (24 ; 124 ; 224 ; 324) est prévu dans la première section de tête (16 ; 116 ; 216 ; 316) et un corps de guidage (26 ; 126 ; 226 ; 326) est prévu sur la deuxième section de tête (22 ; 122 ; 222 ; 322), le corps de guidage (26 ; 126 ; 226 ; 326) étant disposé dans l'évidement (24 ; 124 ; 224 ; 324) de manière mobile par rapport à celui-ci ;
**caractérisé en ce que** l'évidement (24 ; 124 ; 224 ; 324) comprend un contour intérieur et le corps de guidage (26 ; 126 ; 226 ; 326) comprend un contour extérieur, qui sont réalisés de telle sorte que, pendant un pivotement des branches d'articulation (12 ; 112 ; 212 ; 312 ; 18 ; 118 ; 218 ; 318) l'une par rapport à l'autre, l'évidement (24 ; 124 ; 224 ; 324) effectue un mouvement de rotation et de translation par rapport au corps de guidage, lors duquel il est guidé de manière forcée autour du corps de guidage (26 ; 126 ; 226 ; 326) et une section prédéfinie du contour intérieur (64) roule, au moins par phases, sur une section prédéfinie du contour extérieur (66).

2. Ensemble articulé (10 ; 110 ; 210 ; 310) selon la revendication 1, **caractérisé en ce que** le corps de guidage (26 ; 126 ; 226 ; 326) est réalisé en forme de goutte, le corps de guidage (26 ; 126 ; 226 ; 326) comprenant en particulier une zone de tête épaissie (34) et un prolongement (36) se rétrécissant.

3. Ensemble articulé (10 ; 110 ; 210 ; 310) selon l'une des revendications précédentes, **caractérisé en ce que** le corps de guidage (26 ; 126 ; 226 ; 326) est réalisé en forme d'arc et/ou avec une courbure harmonieuse, et notamment un côté intérieur (30) du corps de guidage (26 ; 126 ; 226 ; 326) comprend sensiblement la section prédéfinie du contour extérieur (66).

4. Ensemble articulé (10 ; 110 ; 210 ; 310) selon l'une des revendications précédentes, **caractérisé en ce que** l'évidement (24 ; 124 ; 224 ; 324) est réalisé en forme de cœur.

5. Ensemble articulé (10 ; 110 ; 210 ; 310) selon la revendication 5, **caractérisé en ce que** l'évidement (24 ; 124 ; 224 ; 324) comprend une première chambre (40), en particulier distale, et une deuxième chambre (42), de préférence proximale, une saillie (44) étant formée entre les chambres (40, 42), notamment la saillie (44) comprenant sensiblement la section prédéfinie du contour intérieur (64).

6. Ensemble articulé (10 ; 110 ; 210 ; 310) selon l'une des revendications précédentes, **caractérisé en ce que**, dans une position complètement étirée des branches d'articulation (12 ; 112 ; 212 ; 312 ; 18 ; 118 ; 218 ; 318) l'une par rapport à l'autre, un point de début de la section prédéfinie du contour extérieur (66) touche un point de début de la section prédéfinie du contour intérieur (64) et notamment une partie du contour extérieur est en contact surfacique avec une partie du contour intérieur.

7. Ensemble articulé (10 ; 110 ; 210 ; 310) selon les revendications 5 et 6, **caractérisé en ce que** le corps de guidage (26 ; 126 ; 226 ; 326), dans la position complètement étirée des branches d'articulation (12 ; 112 ; 212 ; 312 ; 18 ; 118 ; 218 ; 318) l'une par rapport à l'autre, est disposé sensiblement dans la première chambre (40), notamment dans une zone inférieure, de préférence distale, de la première chambre (40).

8. Ensemble articulé (10 ; 110 ; 210 ; 310) selon l'une des revendications précédentes, **caractérisé en ce que**, dans une position complètement pliée des branches d'articulation (12 ; 112 ; 212 ; 312 ; 18 ; 118 ; 218 ; 318) l'une par rapport à l'autre, un point de fin de la section prédéfinie du contour extérieur (66) touche un point de fin de la section prédéfinie du contour intérieur (64) et notamment une partie du contour extérieur est en contact surfacique avec une partie du contour intérieur, notamment le corps de guidage (26 ; 126 ; 226 ; 326) étant, dans la position complètement pliée des branches d'articulation (12 ; 112 ; 212 ; 312 ; 18 ; 118 ; 218 ; 318) l'une par rapport à l'autre, disposé sensiblement dans la deuxième chambre (42), notamment dans une zone supérieure, de préférence proximale, de la deuxième chambre (42).

9. Ensemble articulé (10 ; 110 ; 210 ; 310) selon l'une des revendications précédentes, **caractérisé en ce qu'**au moins l'une des sections prédéfinies comprend un contour qui décrit sensiblement une poloïde de l'articulation d'extrémité, notamment la section prédéfinie du contour intérieur (64) décrivant sensiblement une poloïde mobile (499) de l'articulation d'extrémité.

10. Ensemble articulé (10 ; 110 ; 210 ; 310) selon la revendication 9, **caractérisé en ce que** la section prédéfinie du contour extérieur (66) décrit sensiblement une poloïde fixe (500) de l'articulation d'extrémité.

11. Ensemble articulé (10 ; 110 ; 210 ; 310) selon l'une des revendications précédentes, **caractérisé en ce qu'**un élément réduisant la friction, notamment un disque de glissement, est prévu entre la première section de tête (16 ; 116 ; 216 ; 316) et la deuxième section de tête (22 ; 122 ; 222 ; 322).

12. Ensemble articulé (10 ; 110 ; 210 ; 310) selon l'une des revendications précédentes, **caractérisé en ce qu'**au moins un moyen de liaison (28), notamment un boulon (180 ; 280 ; 380), une vis ou un rivet, et/ou un produit auxiliaire sans forme, de préférence un adhésif, fixe le corps de guidage (26 ; 126 ; 226 ; 326) à la deuxième section de tête (22 ; 122 ; 222 ; 322).

13. Ensemble articulé (10 ; 110 ; 210 ; 310) selon l'une des revendications précédentes, **caractérisé en ce qu'**au moins un élément de butée (52, 54, 56) est associé à l'une des branches d'articulation (12 ; 112 ; 212 ; 312 ; 18 ; 118 ; 218 ; 318), ledit élément étant conçu pour coopérer avec un élément de butée correspondant (52, 54, 56) associé à l'autre branche d'articulation (12 ; 112 ; 212 ; 312 ; 18 ; 118 ; 218 ; 318), de sorte que le pivotement des branches d'articulation (12 ; 112 ; 212 ; 312 ; 18 ; 118 ; 218 ; 318) l'une par rapport à l'autre est limité ou peut être limité.

14. Ensemble articulé (10 ; 110 ; 210 ; 310) selon l'une des revendications précédentes, **caractérisé en ce que** l'articulation d'extrémité est une articulation de genou, particulièrement de préférence une articulation de genou humain.

15. Orthèse comprenant au moins un ensemble articulé (10 ; 110 ; 210 ; 310) selon l'une des revendications 1 à 14, dans laquelle notamment ledit au moins un ensemble articulé (10 ; 110 ; 210 ; 310) est disposé sur un côté latéral ou médial de l'articulation d'extrémité, l'orthèse étant de préférence prévue pour comprendre un dispositif de fixation pouvant être associé à la première section de branche (14), et en outre un dispositif de fixation pouvant être associé à la deuxième section de branche (20), les dispositifs de fixation étant conçus pour être fixé à une extrémité, l'orthèse étant prévue en outre ou en variante pour qu'un dispositif de liaison réglable soit prévu au moins sur une section de branche (14, 20), lequel est conçu pour relier un dispositif de fixation associé à la section de branche (14, 20) dans différentes orientations par rapport à la section de branche (14, 20).
